# EUROPEAN PATENT APPLICATION

(11) **EP 1 832 605 A1**
(43) Date of publication of application: **12.09.2007**
(21) Application number: 06110787.6
(22) Date of filing: 07.03.2006
(51) Int. Cl.: C07K 16/28, A61K 39/395, C12N 15/13, C12N 5/16, A61K 47/48, A61P 35/00, A61P 37/00

(54) **Neural cell adhesion molecule (NCAM) binding antibodies**

(71) Applicant: UNIVERSITÄT ZU KÖLN, 50923 Köln (DE); Medizinische Hochschule Hannover, 30625 Hannover (DE)
(72) Inventor: Jensen, Markus, Frechen 50226 (DE); Klehr, Martin, Köln 50937 (DE); Mühlenhoff, Martina, Hannover 30171 (DE); Berthold, Frank, Köln 50933 (DE)
(74) Representative: Viering, Jentschura & Partner

(57) **Abstract**

The present invention relates to immunoglobulin molecules specific for neural cell adhesion molecule (NCAM) and fragments, variants and derivatives thereof. The present invention further includes pharmaceutical and diagnostic compositions containing them, nucleic acid molecules encoding them, methods for their production and their diagnostic and therapeutic use.

## Description

### Field of the invention

The present invention is in the field of immunology and medicine and relates to immunoglobulin molecules specific for neural cell adhesion molecule (NCAM) and fragments, variants and derivatives thereof. The present invention further relates to pharmaceutical and diagnostic compositions containing immunoglobulin molecules as described herein, methods for their production and their diagnostic and therapeutic use.

### Background of the invention

The neural cell adhesion molecules (NCAMs) are a family of closely related cell surface glycoproteins belonging to the immunoglobulin superfamily and are thought to play an important role in embryogenesis and development (Cunningham et al., Science 236:799-806, 1987). NCAM binds to itself (cis- and trans-homophilic binding) and to a series of counter-receptors, including the fibroblast growth factor receptor (FGFR), other adhesion molecules, and various extracellular matrix components (heterophilic binding). The multiple cis- and trans-homophilic interactions NCAM is involved in facilitate cell-cell adhesion through the formation of zipper-like NCAM-complexes. Some of the heterophilic interactions of NCAM are mutually exclusive, and some interfere with or are dependent on homophilic NCAM interactions. However, both homo- and heterophilic interactions are modulated by posttranslational modifications of NCAM.

NCAM is a single copy gene consisting of at least 24 exons. Alternative splicing and the use of different polyadenylation sites produce a variety of isoforms (Barthels et al. Europ. J. Neurosci. 4:327-337, 1992; Walsh and Dickson Bioassays 4:83-88, 1989). Extensive studies on NCAM expression showed that the expression of the various NCAM isoforms is developmentally and spatially regulated (Moore et al. J. Cell. Biol. 105:1377-1386, 1987; Nybroe et al. Neurochem. Int. 12:251-262, 1988; Edelman, Ann. Rev. Biochem. 54:135-169, 1985; Walsh, Neurochem. Int. 12:263-267, 1988).

Four major isoforms of NCAM have been described in the mouse brain. The two largest isoforms of 180 and 140 kDa are transmembrane proteins, which differ primarily in the length of the cytoplasmatic tail, whereas the 120 kDa isoform is linked to the membrane via a glycosyl-phosphatidylinositol (GPI) anchor. The smallest isoform of 115 kDa is believed to be a secretory protein (Gower et al. Cell 55:955-964, 1988). In the human brain, an isoform of 170 kDa is present, in addition to those present in mouse brain (Bhat et al. Neurochem. Int. 13:487-491, 1988). NCAM isoforms have also been studied in detail in human skeletal muscle. In this instance, a transmembrane protein of 140 kDa and two GPI linked isoforms of 125 and 155 kDa are present. The 115 kDa secretory isoform is also present.

The extracellular part of the NCAM molecule encodes N-terminal 5 Ig-like domains (exons 1-10) and 2 fibronectin type-III homologous repeats (exons 11-13) which are located in the membrane-proximal "stem" region. Variations within the extracellular polypeptide chain are due to alternative usage of small extra exons within the exon borders 7/8 and 12/13. A model for NCAM homophilic binding has been proposed in which the Ig domains bind in a pairwise anti-parallel manner such that Ig I binds Ig V, Ig II binds Ig IV, and Ig III binds Ig III (Ranheim et al. Proc. Natl. Acad. Sci. USA 93:4071-4075, 1996).

NCAM plays an important role in cell-cell interactions, accounting for its role in neuronal differentiation, synaptic plasticity, tissue pattern formation, and neuronal network formation, and the heterophilic NCAM-interactions have been demonstrated to initiate several intracellular signal transduction pathways ultimately leading to biological responses involving cellular differentiation, proliferation, migration and survival. In view of these findings, it has been speculated that it may be also important in disease, especially tumor development and progression and neurodegenerative disorders.

Both homo- and heterophilic NCAM-interactions can be mimicked by synthetic peptides that can induce NCAM-like signalling. *In vitro* and *in vivo* studies suggest that such NCAM mimetics may be used for the treatment of neurodegenerative disorders.

NCAM has also been described as a receptor for glial cell line-derived neurotrophic factor (GDNF). This finding solved a long-standing question regarding RET-independent GDNF signaling, and revealed a novel pathway distinct from both GDNF-RET and NCAM-NCAM signaling. Functional assays of Schwann cell migration and axon growth of CNS neurons suggested physiological significance for this GDNF-NCAM pathway.

NCAM expression has been consistently documented on almost all small cell lung cancer (SCLC) cell types. NCAMs are also occasionally found on other lung cancer cell types (Aletsee-Ufrecht et al. FEBS Lett. 267:295-300, 1990; Rygaard et al. Br. J. Cancer 65:573-577, 1992; Kibbelaar et al., J. Pathol. 159 :23-28, 1989), yet, in such cases, expression of NCAMs is associated with a neuroendocrine phenotype (Carbone et al., Cancer Res. 51:6142-6149, 1991). NCAMs are also expressed on numerous tumor cells expected to be of neuroendocrine origin. These tumor cells include neuroblastoma, brain tumor, rhabdomyosarcoma, nephroblastoma (Wilms' tumor), carcinoid and pituitary tumor cells (Roth et al., Am. J. pathol. 133:227-240, 1988; Figarella-Branger et al. Cancer Res. 50:6364-6370, 1990; Patel et al. Biochem. Soc. Trans. 18:408-410, 1990).

Cytogenetically, the NCAM gene is located at chromosome 11q23. At this location chromosome breaks are frequently observed in various leukemias. In accordance with these findings, NCAM is highly expressed in acute non-lymphocytic leukemia (ANLL), and especially in acute monocytic (AMoL) and acute megakaryocytic leukemia (AMKL) (Ikushima et al. Int. J. Hematol. 54:395-403, 1991). It has been furthermore demonstrated that NCAMs are expressed in multiple myelomas, myeloid and large granular lymphocytic leukemias (Leo et al. Ann. Hematol. 64:132-139, 1992; Griffin et al. J. Immunol. 130:2947-2951, 1983).

An NCAM isoform, termed CD56, is also expressed on natural killer (NK) cells. It has been found that the NK activity is generally correlated with the amount of CD 56 expression. However, the actual function of the CD 56 antigen on these cytotoxic cells is unknown.

NK cells are part of the innate immunity, building the first defence line against pathogens or aberrant cells. Innate immune cells are generally classified as either NK cells or antigen presenting cells (APCs). Both act immediately without prior sensitisation after specialised cell surface receptors have recognized certain characteristic molecular patterns (pathogen associated molecular patterns) shared by various pathogens. In contrast, adaptive immune responses by T and B cells need clone-specific receptor mediated signals leading to clonal expansion followed by efficient effector function usually reached at the peak of immune response many hours to days later. Once initiated, adaptive immunity is highly specific and builds up a long persisting memory that can act fast and efficient upon antigen re-challenge. Innate and adaptive immunity complement one another building a complex defence system and are closely linked together on different functional levels.

Natural killer cells have been hypothesized to play a role in the onset autoimmune disorders. Interestingly, the activity of NK cells correlates to the CD56 (NCAM) expression. Although the specific role of NCAM in autoimmune disorders has not been thoroughly investigated yet, it is assumed that an aberrant increased NK activity due to an increased NCAM expression accounts for the autoimmune reaction. In contrast, an inhibition of the NK cell activity, for example, by a decreased NCAM expression or an impaired NCAM function can promote the development of tumors. Likewise, reduced NK cell activity diminishes the tumor-specific B cell responses. The promotion of inflammatory reactions caused by activated monocytes in rheumatoid arthritis has been demonstrated to involve activated NK cells with high CD56 expression (Pridgeon et al. Rheumatology (Oxford) 42:870-878, 2003; Tak et al. Arthritis Rheum. 37:1735-1743, 1994; Dalbeth et al. Arthritis Rheum. 46:1763-1772, 2002; Dalbeth et al. J. Immunol. 173:6418-6426, 2004).

Ikushima et al. (Int. J. Hem. (1991) 54, 395-403) used a set of monoclonal antibodies, *inter alia* Leu-19, a commonly available murine monoclonal antibody directed against CD56, to determine marker molecules on various hematopoietic and non-hematopoietic malignant cells.

Sakamoto et al. (Eur. Surg. Res. (1994) 26, 230-239) studied the expression of NCAM in the digestive system with murine monoclonal antibody NE-150 raised against a 140 kDa isoform of NCAM.

Roguska et al. (Proc. Natl. Acad. Sci. USA (1994), 91, 969-973) reported the humanization of two murine monoclonal antibodies N901 (anti-CD56) and anti-B4 (anti-CD19) by a process called "resurfacing". In this study it was demonstrated that the humanization of the murine N901 antibody did not impair the affinity of the antibody for CD56.

Lashford et al. (NCI Monographs (1987) 3, 53-57) used murine monoclonal anti-CD56 antibody UJ13A radiolabelled with ¹³¹I for the treatment of neuroblastoma. However, therapeutic benefit could only be demonstrated in one patient, while severe side effects of the used antibody were observed in others.

Jones et al. (NCI Monographs (1987) 3, 125-130) used murine monoclonal anti-CD56 antibody UJ13A radiolabelled with ¹³¹I to determine the pharmacokinetics of the antibody in patients and animal models. The results demonstrated that uptake of the antibody is extremely low, <0.005 %. The authors concluded that while the antibody may be sufficient for diagnosis the studies indicate that it is not suitable for therapeutic purposes.

Goldman et al. (Journal of Pediatrics (1984), 105 (2), 252-256) used the radiolabelled murine monoclonal anti-CD56 antibody UJ13A for the immunolocalization of neuroblastoma and suggest said antibody for diagnostic purposes.

Roy et al. (Journal of the National Cancer Institute (1996), 88(16), 1136-1145) used murine monoclonal anti-CD56 antibody N901 coupled to ricin to eliminate NCAM-expressing tumor cells. The authors could demonstrate that N901-blocked ricin eliminates in a concentration- and time-dependent fashion several logs of both neuroblastoma and small cell lung cancer cells from an excess of normal bone marrow cells in cell culture.

Bourne et al. (Journal of Neuro-Oncology (1991) 10, 111-119) reported that a murine monoclonal antibody raised against retinoblastoma, ERIC-1, recognizes NCAM expressed on brain and tumors arising from the neuroectoderm.

In view of the above, there still exists a need for immunoglobulins that specifically target NCAM (CD56) and which are also suitable, for example, for diagnosis or treatment of NCAM-related malignancies and autoimmune disorders.

The references discussed throughout this document are set forth merely for the information described therein prior to the filing dates of this document, and nothing herein is to be construed as an admission, either express or implied, that the references are "prior art".

### Summary of the invention

It is an object of the present invention to provide such molecules. Accordingly, the present invention provides monoclonal and recombinant antibodies and fragments thereof directed against epitopes of human NCAM (CD56). These antibodies are suitable for the diagnosis and/or treatment of NCAM-related malignancies and autoimmune disorders in humans.

Thus, in a first aspect the invention relates to an immunoglobulin molecule. This molecule includes at least one immunoglobulin heavy chain or fragment thereof which comprises a variable domain comprising in sequence the hypervariable regions CDR-H1, CDR-H2, and CDR-H3, wherein said CDR-H1 has the amino acid sequence set forth in SEQ ID NO:12, said CDR-H2 has the amino acid sequence set forth in SEQ ID NO:14, and said CDR-H3 has the amino acid sequence set forth in SEQ ID NO:16. This immunoglobulin molecules further includes one immunoglobulin light chain or fragment thereof which comprises a variable domain comprising in sequence the hypervariable regions CDR-L1, CDR-L2, and CDR-L3, wherein said CDR-L1 has the amino acid sequence set forth in SEQ ID NO:6, said CDR-L2 has the amino acid sequence set forth in SEQ ID NO:8, and said CDR-L3 has the amino acid sequence set forth in SEQ ID NO:10. This immunoglobulin molecule has specificity for an epitope of human NCAM.

In one embodiment of the invention, said NCAM epitope which is bound by the immunoglobulin of the invention is arranged in the third Ig-like domain (Ig-like domain III) of NCAM, particularly within the stretch of amino acids 255-304 of NCAM. However binding of antibodies of the invention is not restricted exclusively to the third Ig like domain but can include other epitopes on the NCAM molecule as well.

In one embodiment of the invention the variable domain of an immunoglobulin heavy chain and the variable domain of an immunoglobulin light chain are covalently linked to each other by a peptide linker connecting either the N-terminus of the light chain with the C-terminus of the heavy chain or vice versa. The linker has typically a length of about 4 to about 30 or of about 5 to about 25 amino acids. The length of the linker determines the characteristics of the resulting molecules. Linker length of about 15 amino acid residues or more result generally in the formation of single chain Fv fragments. If the linker is only about 8-12 or less amino acids long, such immunoglobulin constructs may dimerize or, in case of a linker length lower than 5 amino acids, multimerize to form, for example, diabodies or triabodies.

In another embodiment of the invention, the at least one immunoglobulin heavy chain further comprises the constant part or fragment thereof of a human heavy chain and the at least one immunoglobulin light chain further comprises the constant part or fragment thereof of a human or murine light chain.

In a further embodiment of the present invention, the invented immunoglobulin molecule includes at least one heavy chain and at least one light chain. The at least one heavy chain comprises a variable domain comprising the amino acid sequence shown in SEQ ID NO:2 and the constant domain of a human heavy chain, whereas the at least one light chain, which comprises a variable domain comprising the amino acid sequence shown in SEQ ID NO: 4 and the constant domain of a human or murine light chain.

Also encompassed by the present invention are fragments, variants and derivatives of the above immunoglobulin molecule, as long as they retain the function to specifically bind an epitope of human NCAM.

Also encompassed in the present invention are immunoglobulin molecules as detailed above, wherein the constant domain or fragment thereof of the human heavy chain is of the γ1 type and the constant domain or fragment thereof of the human light chain is of the κ type.

In still other embodiments of the invention, the immunoglobulin molecules as detailed above may comprise the constant domain or fragment thereof of a human heavy chain, preferably of the γ1 type, and a constant domain or fragment thereof of a murine light chain, preferably of the κ or λ type.

In a preferred embodiment, the immunoglobulin molecules of the invention comprise two light chains and two heavy chains, with the two light chains and the two heavy chains being preferably identical to each other.

In another preferred embodiment, the immunoglobulin molecules of the invention comprise murine variable domains and human constant regions, thus forming chimeric humanized immunoglobulin molecules. The murine variable parts are in case of the heavy chain preferably of the γ1 type and in case of the light chain preferably of the κ or λ type. The human Fc part is fully functional, meaning capable of interacting with human Fc receptors.

In a further embodiment, the immunoglobulin molecules of the invention comprise murine complementarity determining regions (CDRs), whereas the rest of the molecule is of human origin, thus forming a humanized immunoglobulin molecule. In addition to the murine CDRs, parts of the framework regions of the variable domain can also be of murine origin.

The immunoglobulin molecule according to the invention may have an affinity (dissociation constant K_{D}) for NCAM of at least 1 x 10⁻⁶ M⁻¹, preferably 1 x 10⁻⁷ M⁻¹ or better. The determination of K_{D} values is used in the art as a measure to describe the strength of binding between two binding partners. The dissociation constant, K_{D}, relates to the ratio between the concentrations of free, uncomplexed binding partners, and the complex at equilibrium conditions. It follows that the smaller K_{D}, the stronger the binding. As will be further detailed below in the Examples section, the dissociation constant K_{D} of the antibodies may be determined by radiolabelling the antibodies, using NCAM expressing cells as binding partners, measuring cell specific radioactivity, and determining the K_{D} of radio-iodinated antibodies using the scatchard plot method.

In one embodiment of the invention an immunoglobulin molecule of the invention may be radiolabelled, with the radiolabel preferably selected from the group consisting of ³²P, ⁶⁷Cu, ⁹⁰Y, ¹¹¹In, ¹²⁵I, ¹³¹I, ¹³⁶Re, ¹⁸⁸Re, ¹⁹⁸Au, and ²¹¹At.

In a further embodiment, the invention includes immunoglobulin molecules which are conjugated to a toxin. Examples of suitable toxins include, but are not limited to, maytansine, N^{2'}-deacetyl-N^{2'}- (3-mercapto-1-oxopropyl)-maytansine, ricin, abrin, ribonuclease, DNase I, Staphylococcal enterotoxin-A, pokeweed antiviral protein, gelonin, diphtherin toxin, *Vinca* alkaloids, doxorubicin, methotrexate, *Pseudomonas* endo- and exotoxin or derivatives and pharmaceutically acceptable salts thereof. Presently preferred in one embodiment are maytansine and its derivatives.

In a second aspect, the invention is directed to a monoclonal antibody, termed 5A2, obtainable from a hybridoma which secretes anti-NCAM antibody, said hybridoma being identified by deposit number DSM ACC2757. This antibody is of the IgG1 type, and comprises a chimeric heavy chain comprising the constant part of a human heavy chain and a murine variable domain, and a fully murine light chain. Also encompassed in the present invention are fragments, variants and derivatives of the monoclonal antibody 5A2. The monoclonal antibody 5A2 and fragments thereof may also be radiolabelled or conjugated to a toxin as described above for the immunoglobulin molecules of the invention.

Also encompassed by the present invention is the use of these monoclonal antibodies or immunoglobulin molecule as a medicament.

In a third aspect, the present invention relates to a pharmaceutical composition comprising at least one immunoglobulin molecule or monoclonal antibody according to the invention and optionally a pharmaceutically acceptable carrier. Additionally, the compositions of the invention can further comprise an excipient, auxiliary, preservative, buffer agent, solvent or the like, all of which are known to a person skilled in the pharmaceutical art.

In a fourth aspect, the present invention is directed to the use of the compositions of the invention for the manufacture of a pharmaceutical for the diagnosis, treatment and/or prevention of NCAM-related malignancies and autoimmune disorders in a subject. In some embodiments the subject is preferably a mammal, more preferably a human.

The NCAM-related malignancy may be of neuroendocrine origin. In some embodiments, the NCAM related malignancies include, but are not limited to, neuroblastoma, small cell lung cancer, brain tumor, rhabdomyosarcoma, nephroblastoma (Wilms' tumor), carcinoid, pituitary tumor, acute non-lymphocytic leukemia (ANLL), acute monocytic leukemia (AMoL), acute megakaryocytic leukemia (AMKL), multiple myeloma, myeloid leukemia, insulinoma and large granular lymphocytic leukemia. In one presently preferred embodiment the malignancy is a neuroblastoma. An example of an autoimmune disorder that can be treated in accordance with the invention is rheumatoid arthritis.

If the compositions of the invention are used for therapeutic purposes, for example to treat a tumor, they can be combined with a chemotherapeutic agent. Suitable examples of chemotherapeutic agents include, but are not limited to, cyclophosphamide, etoposide, vincristine, procarbazine, prednisone, carmustine, doxorubicin, methotrexate, bleomycin, dexamethasone, phenyl butyrate, bryostatin-1 and leucovorin.

The treatment with the chemotherapeutic agent may not be concomitantly. Accordingly, radiation therapy or chemotherapy may precede or follow the administration of compositions according to the invention.

Also encompassed in the invention is a hybridoma which secretes the monoclonal anti-NCAM antibody 5A2 of the invention, said hybridoma being identified by deposit number DSM ACC2757.

In a further aspect, the present invention relates to nucleic acid molecules encoding the immunoglobulin molecules of the invention. Thus, the present invention also provides DNA molecules encoding a single domain NCAM-binding molecule of the invention, a single chain NCAM-binding molecule of the invention, a heavy or light chain or fragments thereof of a NCAM-binding molecule of the invention. Also included are expression vectors comprising, in addition to at least one nucleic acid sequence encoding an immunoglobulin molecule according to the invention, a suitable promoter and/or optionally genes encoding immunoglobulin heavy and light chain constant parts.

In exemplary embodiments, the nucleic acid molecules of the invention may comprise the nucleic acid sequences set forth in SEQ ID Nos. 1, 3, 5, 7, 9, 11, 13, and 15. In a presently preferred embodiment, a nucleic acid molecule of the invention encodes an immunoglobulin heavy chain or fragment thereof and comprises the nucleic acid sequences set forth in SEQ ID Nos. 11, 13, and 15 or the nucleic acid sequence set forth in SEQ ID NO: 1. In another presently preferred embodiment, a nucleic acid molecule of the invention encodes an immunoglobulin light chain or fragment thereof and comprises the nucleic acid sequences set forth in SEQ ID Nos. 5, 7, and 9, or the nucleic acid sequence set forth in SEQ ID NO: 3.

Also encompassed by the present invention is the use of the DNA molecules of the invention for the production of an NCAM-binding molecule of the invention by recombinant means. Thus, an NCAM-binding molecule of the invention may be produced by recombinant DNA techniques. A recombinant cell containing a vector or nucleic acid molecule of the invention is a further aspect of the invention.

Also intended to fall within the scope of the invention is the production of the desired antibody in a cell culture or in a transgenic animal. A suitable transgenic animal may be obtained according to standard methods.

The advantageous properties of the immunoglobulins of the invention, namely high specificity and affinity as well as suitability for therapeutic purposes, i.e. the treatment of NCAM-related malignancies, have been demonstrated for the first time by the present invention, and will be further illustrated by the following detailed description in conjunction with the non-limiting experimental examples and the figures.

### Brief description of the drawings

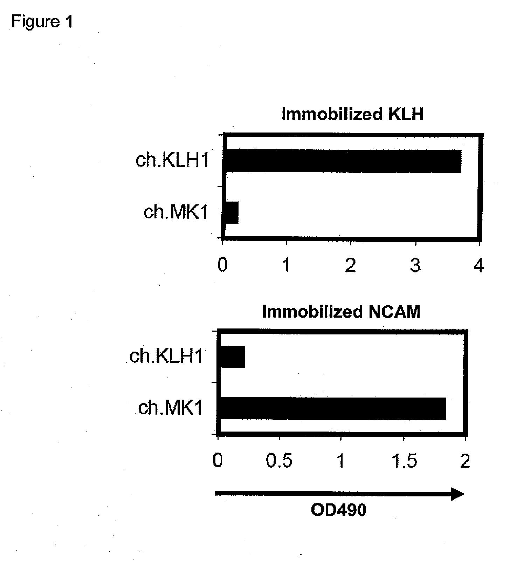

Figure 1 shows the specific binding of ch.MK1 antibodies to immobilized antigen as demonstrated via ELISA, ch.KLH1 is a humanized control antibody raised against keyhole limpet hemocyanine (KLH). Purified NCAM (in its polysialated form)and purified keyhole limpet hemocyanine (KLH) as a control were immobilized on 96 well plastic plates. Bound antigen were detected with ch.MK1 or ch.KLH Abs respectively and anti-human Ig-POD secondary antibodies.

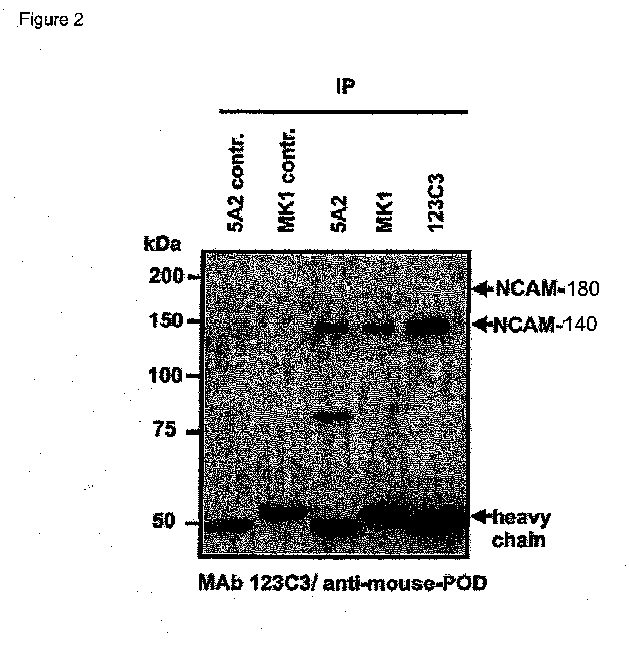

Figure 2 shows the specific binding of chimeric monoclonal ch.MK1 and monoclonal 5A2 antibodies to native NCAM. NCAM molecules were precipitated from IMR-5 (NCAM+ neuroblastoma) cell lysates by ch.MK1 or 5A2 Abs plus Protein A sepharose beads. Precipitated NCAM was then analysed in a Western Blot and NCAM bands were detected using the murine reference antibody 123C3. Cell lysates were pretreated with neuraminidase to digest polysialic acid substitutes that could weaken the precipitation step. Abbreviations used: 5A2 contr., immunoprecipitation with 5A2 Abs without cell lysate as a control; MK1 contr., immunoprecipitation with ch.MK1 Abs without cell lysate as a control; 5A2, immunoprecipitation with 5A2 Abs with cell lysate; MK1, immunoprecipitation with ch.MK1 Abs with cell lysate; 123C3, immunoprecipitation with 123C3 Abs with IMR-5 cell lysate.

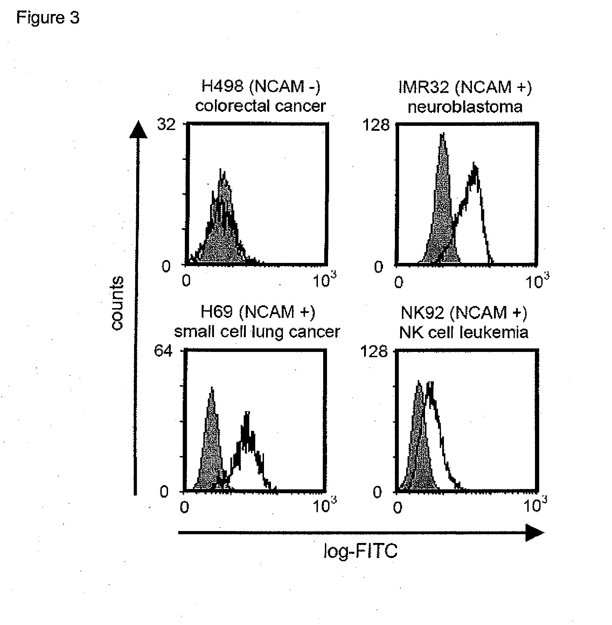

Figure 3 presents results that demonstrate that ch.MK1 can react with NCAM on viable cells. IMR-32 (NCAM+ neuroblastoma), H69 (NCAM+ small cell lung cancer), NK92 (NCAM+ NK cell leukaemia) and H498 (NCAM- colorectal cancer) cells were incubated with ch.MK1 or Trastuzumab (anti-Her2(neu) chimeric Abs as a control. Abs binding to the cells were detected by goat anti-human IgG-biotin and streptavidin-FITC (fluorescein isothiocyanate). The results clearly demonstrate binding of ch.MK1 Abs to NCAM on cell surfaces.

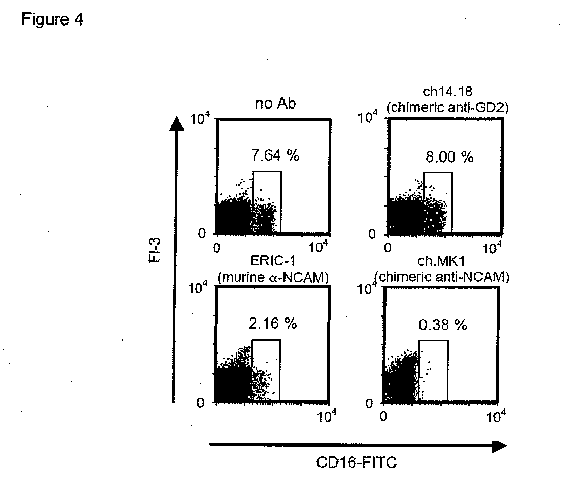

Figure 4 demonstrates that ch.MK1 Abs are capable of depleting natural killer (NK) cells. Freshly isolated PBMC (peripheral blood mononuclear cells) were incubated with ch.MK1, ERIC-1 (murine anti-human-NCAM antibody), ch14.18 (chimeric anti-GD2 antibody; not reacting with NK cells), or without Abs as a control. Cells were harvested after seven days, stained with anti-CD16-FITC Abs and analysed by flow cytometry. Four independent experiments were performed, two of them with addition of 100 U/ml human IL-2 as a control to prevent spontaneous NK cell apoptosis, revealing the same results.

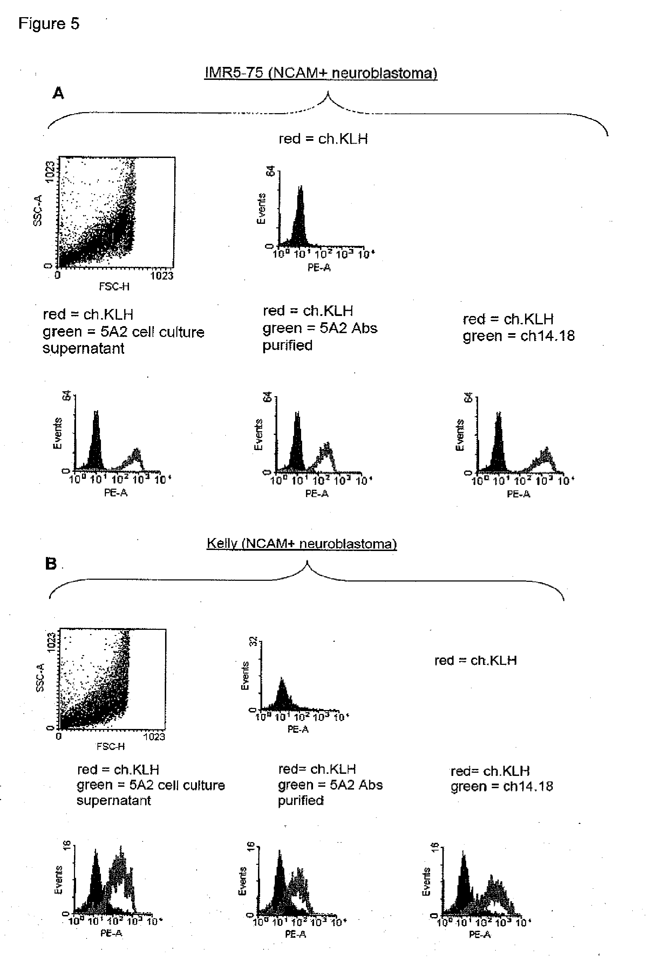

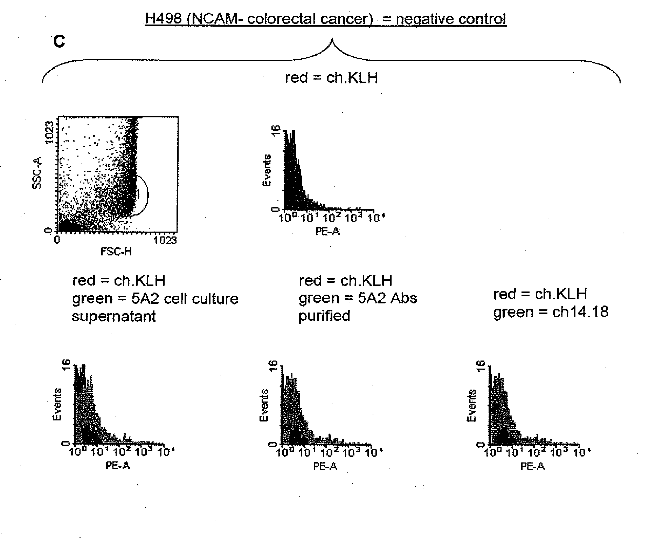

Figure 5 shows that murine monoclonal anti-NCAM antibody 5A2 specifically binds to NCAM on viable cells. IMR5-75 (NCAM+ neuroblastoma) (**A**), Kelly (NCAM+ neuroblastoma) (**B**) and H498 (NCAM- colorectal cancer) cells (**C**) were incubated with 5A2 or ch.KLH Abs as a control. Abs reacting with cells were detected by goat anti-human IgG-biotin and streptavidin-PE. The results clearly demonstrate binding of 5A2 Abs to NCAM on cell surfaces.

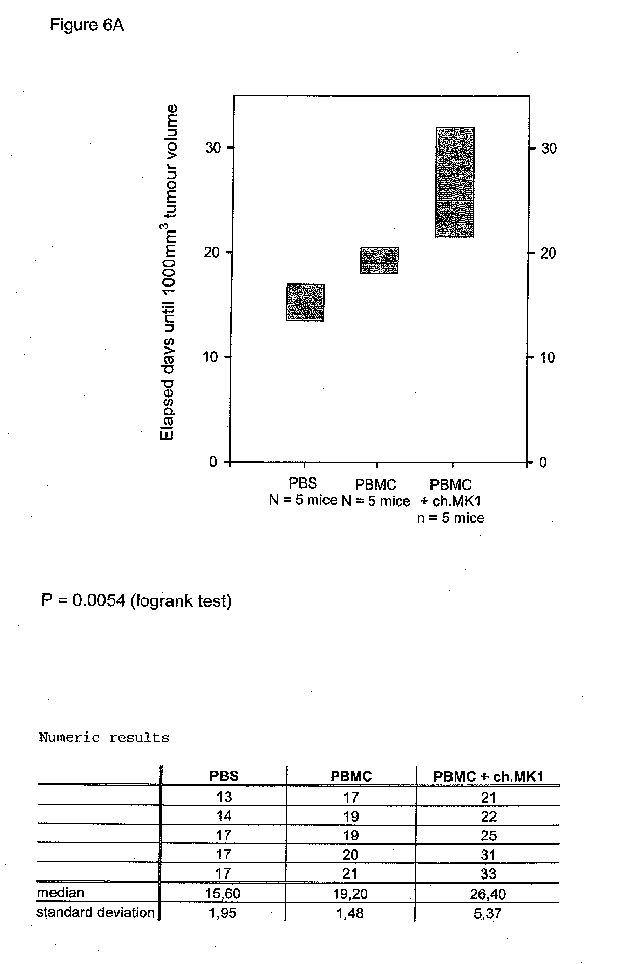

Figure 6A shows the inhibitory effect of anti-NCAM Ab ch.MK1 on subcutaneous IMR5-75 tumor growth in SCID mice. Prior s.c. injection of IMR5-75 cells mice were treated i.v. with anti-ASGM1 (asialo-GM1) antiserum to deplete murine NK cells in order to synchronize tumour growth. 15 SCID mice were subcutaneously challenged with 2 x 10⁷ IMR5-75 NCAM expressing human neuroblastoma cells to induce subcutaneous growth of a tumor nodule. At day 9 tumor growth rate was 100 %. Mice were treated at day 9, 10, 13, 14, 16 and 17 with subcutaneous injection of 2x10⁷ freshly isolated human PBMC plus 200 µg ch.MK1 Abs or PBMC alone or PBS only (phosphate buffered saline) as a control. Tumor growth was monitored by three-dimensional calliper measurement and tumor volume was calculated (volume [mm³] =4/3*3.14159*x/2*y/2*z/2). Endpoint was 1000 mm³ tumor volume. Box plots show median value and range of each group. P = 0.0054 (logrank test).

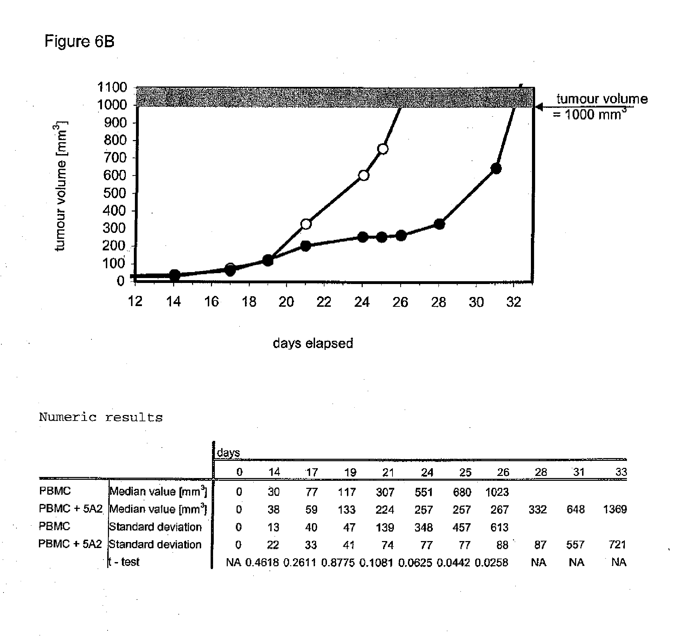

Figure 6B shows the inhibitory effect of anti-NCAM Abs on subcutaneous IMR5-75 tumor growth in SCID mice. Prior s.c. injection of IMR5-75 cells mice were treated i.v. with anti-ASGM1 (asialo-GM1) antiserum to deplete murine NK cells in order to synchronize tumour growth. Groups of 6 SCID mice were subcutaneously challenged with 2 x 10⁷ IMR5-75 NCAM expressing human neuroblastoma cells to induce subcutaneous growth of a tumor nodule. At day 14 mice were treated intravenously with 2 x 10⁷ freshly isolated human PBMC plus 100 µg 5A2 Abs (closed circles) or PBMC alone (open circles) as a control. Treatment was intensified on day 19, 21, and 24 by injecting 2 x 10⁷ freshly isolated human PBMC plus 100 µg 5A2 Abs intravenously and an additional intralesional injection of the same doses. Tumor growth was monitored by three-dimensional measurement by calliper and tumor volume was calculated (volume [mm³] =4/3*3.14159*x/2*y/2*z/2). From 6 mice per group 5 mice per group entered the analyses and one mouse per group was censored as an outlier. At day 25 and 26 tumor sizes differed significantly between experimental and control group using the students t-test to compare tumor sizes at each time point.

### Detailed description of the invention

The present invention relates to immunoglobulin molecules binding to neural cell adhesion molecule (NCAM). By "NCAM binding molecule" or "CD56 binding molecule" is meant any molecule capable of binding to the NCAM (CD56) antigen either alone or associated with other molecules. The binding reaction may be shown by standard methods (qualitative assays) including, for example, a bioassay for determining the inhibition of homo- or heterophilic NCAM binding or any kind of binding assays, with reference to a negative control test in which an antibody of unrelated specificity, e.g. an anti-KLH antibody, is used. Also encompassed by the present invention are fragments, variants and derivatives of the above immunoglobulins.

An "antigen" is a molecule or a portion of a molecule capable of being bound by an antibody. The antigen (alone, or in order to increase its immunogenicity, together with a suitable adjuvant) is additionally capable of inducing an animal to produce antibody capable of binding to an epitope of that antigen. An antigen may have one or more than one epitope. The specific reaction referred to above is meant to indicate that the antigen will react, in a highly selective manner, with its corresponding antibody.

In one embodiment of the invention, the immunoglobulin molecules directed against human NCAM are antibodies, preferably monoclonal antibodies.

The term "antibody" (also abbreviated "Ab") as used herein is meant to include a polyclonal or monoclonal antibody, and fragments and regions thereof, as well as derivatives thereof, which are capable of binding portions of a target antigen. Anti-NCAM antibodies of the present invention include at least one heavy chain variable region and/or light chain variable region that binds to NCAM, and optionally further include at least one heavy chain constant region and/or a light chain constant region.

Generally, intact antibodies are composed of two light chains and two heavy chain molecules; these chains form a general "Y" shape, with both light and heavy chains forming the arms of the Y and the heavy chains forming the base of the Y.

Light and heavy chains are divided into domains of structural and functional homology. The variable domains of both the light ("VL") and the heavy ("VH") chains form the Fv fragment and determine recognition and specificity. The constant region domains of light ("CL") and heavy ("CH") chains confer important biological properties, e.g. antibody chain association, secretion, transplacental mobility, Fc receptor binding complement binding, etc.

The variable domains of the heavy and light chains have the same basic structure consisting of antigen binding regions and framework regions. More particularly, the antigen binding characteristic of an antibody is essentially determined by 3 specific regions in the variable domain of the heavy and light chains, the hypervariable regions or complementarity determining regions (CDRs). These 3 hypervariable regions alternate with 4 framework regions (FRs), whose sequences are relatively conserved and which are usually not directly involved in binding of the antigen. The CDRs form loops and are held in close proximity by the framework regions which largely adopt a beta-sheet conformation. The CDRs of a heavy chain together with the CDRs of the associated light chain essentially constitute the antigen-binding site of the antibody molecule. The determination as to what constitutes an FR or a CDR region is usually made by comparing the amino acid sequence of a number of antibodies raised in the same species.

The series of events leading to immunoglobulin gene expression in the antibody producing cells are complex. The variable domain region gene sequences are located in separate germ line gene segments referred to as "'VH" , "D" , and "JH", or "VL" and "JL." These gene segments are joined by DNA rearrangements to form the complete V regions expressed in heavy and light chains, respectively. The rearranged, joined V segments (VL-JL and VH-D-JH) then encode the complete variable regions or antigen binding domains of light and heavy chains, respectively.

To date, monoclonal antibodies produced by immortalized hybridoma cell lines play an important role in diagnostic and therapeutic applications. Recent advances in monoclonal antibody (mAb) technology including humanization and mAb engineering have facilitated the use of mAbs for the treatment of human cancers with enhanced antitumor activity, for example, Trastuzumab, Gemtuzumab-Ozogamycin, Rituximab and Infliximab. These mAbs selectively target tumor tissues and have been safely administered in cancer patients.

However, a factor hampering the development of monoclonal antibodies directed against tumors lies in the difficulty to obtain antibodies capable of differentiating between cancer and normal tissue cells with high levels of specificity. In addition, most tumors are heterogeneous and a large proportion of the antibodies developed against tumors may target only a fraction of the tumor cells. Thus, the identification of antigens or epitopes highly expressed on tumor cells remains one of the main objectives in current immunology.

Further to the use of monoclonal antibodies alone, which can suffice to elicit an immune response against the targeted tumor cells, such antibodies also offer the possibility to deliver radionuclides, toxins, or other therapeutic agents selectively to tumor sites, thus limiting toxicity to normal tissues.

However, monoclonal antibodies raised against a protein naturally found in all humans must necessarily be developed in a non-human system, e.g. in mice. As a direct consequence of this, a xenogenic antibody as produced by a hybridoma, when administered to humans, may elicit an undesirable immune response, which is predominantly mediated by the constant part of the xenogenic immunoglobulin. For murine-based monoclonal antibodies, this is often referred to as a Human Anti-Mouse Antibody response, or "HAMA" response. Additionally, these "foreign" antibodies can be attacked by the immune system of the host such that they are, in effect, neutralized before they reach their target site. This clearly limits the use of such antibodies, as they cannot be administered over a prolonged period of time. Therefore, it is preferred to use an antibody format that at least partly lacks such immunogenic parts. Examples of suitable antibody molecules are single chain Fv fragments that completely lack constant domains or chimeric or humanized intact antibodies and fragments thereof (such as Fab fragments) which are not likely to elicit a substantial allogenic response when administered to humans.

A further problem with this approach is that non-human monoclonal antibodies (e.g., murine monoclonal antibodies) typically lack human effector functionality, i.e., they are unable to, inter alia, mediate complement dependent lysis or lyse human target cells through antibody dependent cellular toxicity or Fc-receptor mediated phagocytosis.

Thus, therapy in humans requires human antibodies to fully exploit immune effector functions and to prevent anti-immunoglobulin responses. One option to overcome these problems is the utilization of "humanized" or "chimeric" antibodies. Said techniques offer the possibility to transfer the full antigen specificity and binding avidity of murine monoclonal antibodies to a human antibody by grafting the murine variable domains or the murine complementarity determining regions (CDRs) onto a human constant region framework or a human variable region framework, respectively.

Generation of humanized monoclonal antibodies requires genetic modification of a well-established murine monoclonal antibody by exchanging the murine Fc part alone or the Fc part together with the framework regions with human immunoglobulin sequences. Since the CDR Regions of the murine antibody remain untouched with this technology binding specificity and affinity are largely conserved. A more advanced technology is the generation of said humanized monoclonal antibodies by immunizing mice that harbour human immunoglobulin gene fragments. By this technology entirely new Abs can be generated fast and efficiently which are instantly humanized. Simple classical hybridoma technology is required to establish hybridoma cell lines from those mice.

A "single chain antibody (fragment)" consists of only the variable domains of an antibody's heavy and light chains covalently linked by a peptide linker usually consisting of from 4 to 30 amino acids, preferably from 5 to 25 amino acids. Therefore, such a structure does not include the constant part of the heavy and light chains and it is believed that the small peptide spacer should be less antigenic than a whole constant part. Dependent on the linker length, such molecules may dimerize or multimerize to form diabodies, triabodies or even higher multimerized forms.

By "chimeric antibody or antibody fragment" is meant an antibody in which the constant regions of heavy or light chains or both are of human origin while the variable domains of both heavy and light chains are of non-human (e.g. murine) origin.

By "humanized antibody or antibody fragment" is meant an antibody in which, for example, the hypervariable regions (CDRs) are of non-human (e.g. murine) origin, while all or substantially all the other parts of the immunoglobulin e.g. the constant regions and the highly conserved parts of the variable domains, i.e. the framework regions, are of human origin. A humanized antibody may retain a few amino acids of the murine sequence in the parts of the framework regions adjacent to the hypervariable regions. A humanized antibody fragment can, for example, also be a Fab fragment with a completely murine Fv fragment and constant human CH and Cl domains.

Hypervariable regions may be associated with any kind of framework regions, preferably of murine or human origin. Suitable framework regions are described in "Sequences of proteins of immunological interest", Kabat E.A. et al, US department of health and human services, Public health service, National Institute of Health.

### Immunoglobulins

The invention relates to a human NCAM-binding immunoglobulin molecule, which comprises in sequence the hypervariable regions CDR-H1, CDR-H2, and CDR-H3, having the amino acid sequence set forth in SEQ ID NO:12, 14, and 16, and/or the hypervariable regions CDR-L1, CDR-L2, and CDR-L3, having the amino acid sequence set forth in SEQ ID NO:6, 8, and 10.

In one embodiment of the invention, the NCAM epitope the invented immunoglobulin is binding to may lie in the third Ig-like domain (Ig-like domain III) of NCAM, particularly within the stretch of amino acids 255-304 of NCAM.

The term "epitope" is meant to refer to that portion of any molecule capable of being recognized by and bound by an antibody. Epitopes usually consist of chemically active surface groupings of molecules such as amino acids or sugar side chains and have specific three dimensional structural characteristics as well as specific charge characteristics.

In one embodiment of the invention, the hypervariable regions CDR-H1, CDR-H2, and CDR-H3, having the amino acid sequence set forth in SEQ ID NO:12, 14, and 16 are part of the variable domain of an immunoglobulin heavy chain and the hypervariable regions CDR-L1, CDR-L2, and CDR-L3, having the amino acid sequence set forth in SEQ ID NO:6, 8, and 10 form part of the variable domain of an immunoglobulin light chain.

Said variable domain of an immunoglobulin heavy chain comprising the above CDR-H1, CDR-H2, and CDR-H3 region and said variable domain of an immunoglobulin light chain comprising the above CDR-L1, CDR-L2, and CDR-L3, may be covalently linked to each other by a peptide linker, being preferably 4-30, more preferably 5-25 amino acids in length. The above constructs may dimerize or multimerize to form, for example, diabodies and triabodies. The peptide linker may be bound either to the N-terminus of the variable domain of an immunoglobulin heavy chain and to the C-terminus of the variable domain of an immunoglobulin light chain or vice versa.

The above CDRs as well as part of the framework or the complete variable domains may be of murine origin. The murine framework parts of the variable domain may in case of the heavy chain be preferably of the γ type and in case of the light chain preferably of the κ or λ type. Particularly preferred are the γ1 and the κ type.

The variable domain of an immunoglobulin heavy chain comprising the hypervariable regions CDR-H1, CDR-H2, and CDR-H3, having the amino acid sequence set forth in SEQ ID NO:12, 14, and 16, may further comprise the constant part or fragment thereof of a human heavy chain, and the variable domain of an immunoglobulin light chain comprising the hypervariable regions CDR-L1, CDR-L2, and CDR-L3, having the amino acid sequence set forth in SEQ ID NO:6, 8, and 10, may further comprise the constant part or fragment thereof of a human or murine light chain.

One immunoglobulin molecule of the invention may include at least one heavy chain, which comprises a variable domain comprising the amino acid sequence shown in SEQ ID NO:2 and the constant domain of a human heavy chain, and at least one light chain, which comprises a variable domain comprising the amino acid sequence shown in SEQ ID NO:4 and the constant domain of a human or murine light chain.

The constant domain or fragment thereof of the human heavy chain may be of the γ1, γ2, γ3, γ4, µ, α1, α2 , δ, or ε type, whereas the constant domain or fragment thereof of the human or murine light chain may be of the κ or λ type (which includes the λ1, λ2, and λ3 subtypes). Preferably the human heavy chain is of the γ, more preferably of the γ1 type, and the light chain is of the human κ type.

In order to form a typical antibody molecule, the immunoglobulin molecules of the invention may comprise two, preferably identical, light chains and two, preferably identical, heavy chains. Therein, the human Fc part may be fully functional, meaning capable of interacting with human Fc receptors.

Thus, as the CDRs or the complete variable domain of both the heavy chain as well as the light chain may be of murine origin, whereas the constant part of the heavy and light chains is of human origin, the invention features chimeric and fully humanized immunoglobulin molecules. Another aspect of the invention relates to an antibody comprising a chimeric or fully humanized heavy chain, and a fully murine light chain. One of skill in the art would know the parameters for choosing a particular type of anti-NCAM antibody. For instance, chimeric and humanized antibodies are beneficial for decreased immunogenicity, and for facilitating antibody effector mediated immune reactions via the human constant region domains. Murine and other mammalian antibodies, in contrast, are beneficial for delivering a radiolabel to the tumor cell, as such antibodies generally have a decreased half-life in vivo.

The above-mentioned variant, in which the constant region of the light chain is also of murine origin, results in an antibody molecule combining skills of a chimeric antibody and a murine antibody. This antibody variant may have importance for delivering radioactive isotopes to NCAM expressing target cells.

The preferred NCAM binding molecules of the invention are chimeric monoclonal anti-NCAM antibodies in order to overcome limitations encountered with murine antibodies, including short half-life, limited ability to stimulate human effector functions, and immunogenicity. Although the present invention relates primarily to chimeric murine/human antibodies, the murine part can originate from any other species as well. Said species can for example be selected from a rat, a rabbit, a goat, a sheep etc., all of which are well known to a person skilled in the art.

Also encompassed in the present invention are fragments, variants and derivatives of the above immunoglobulin molecule, as long as they retain the function to specifically bind human NCAM epitopes.

As it is well-known, minor changes in an amino acid sequence such as deletion, addition or substitution of one or several amino acids may lead to an allelic form of the original protein, which has substantially identical properties. It is therefore understood that the skilled person having at hand the sequence information provided herein may carry out modifications to the immunoglobulin molecules of the invention in order to optimise their properties. For example, based on the sequence of the variable domains described here, a three-dimensional model of the antibody binding site (the paratope) can be created by molecular modelling in order to identify promising positions in the paratope to further improve the interaction with the epitope. Mutants identified by such a rationale modelling approach can then be generated by site-directed mutagenesis. Another approach to further improve the interaction of the antibody molecules disclosed herein is to use evolutionary methods such as phage display.

In such evolutionary methods, the coding sequence of the variable regions, in particular of the CDRs can serve as a starting point for (random) mutagenesis of an antibody molecule of the present invention. For the mutagenesis of the amino acids in the CDR loops (so creating a naive nucleic acid library), the person skilled in the art has at his disposal various known methods for site-directed mutagenesis or for mutagenesis by means of the polymerase chain reaction. The mutagenesis method can, for example, be characterized in that mixtures of synthetic oligodeoxynucleotides, which bear a degenerate base composition at the desired positions, can be used for introduction of the mutations. The use of nucleotide building blocks with reduced base pair specificity, as for example inosine, is also an option for the introduction of mutations into the chosen sequence segment or amino acid positions. A further possibility is the so-called triplet-mutagenesis. This method uses mixtures of different nucleotide triplets each of which codes for one amino acid for the incorporation into the coding sequence.

After having brought the coding nucleic acid sequences that were subjected to mutagenesis to expression, for example, as a scFV or Fab fragment, the clones carrying the genetic information for the plurality of respective antibody fragments which bind NCAM can be selected from the library obtained. Known expression strategies and selection strategies can be employed for the selection of these clones. Methods of this kind have been described in the context of the production or the engineering of recombinant antibody fragments, such as the "phage display" technique ( Hoess, Curr. Opin. Struct. Biol. 3 (1993), 572-579; Wells and Lowman, Curr. Opin. Struct. Biol. 2 (1992), 597-604) or "colony screening" methods (Skerra et al., Anal. Biochem. 196 (1991), 151-155) or "ribosome display" (Roberts, Curr. Opin. Chem. Biol. 3 (1999) 268-273).

It is thus intended that modifications of the amino acid sequences of the CDRs in antibody molecules that are based on the CDR sequences disclosed herein are within the scope of the present invention, as long as the resulting antibodies retain their ability to bind NCAM at least to substantially the same extent as a molecule having the starting amino acid sequence. Accordingly, antibody molecules that are based on the amino sequences of the CDR regions disclosed herein and that show an increased binding compared to the exemplary molecules illustrated here are also encompassed in the present invention. It is noted in this context that it is well-known in the art that, for example, the CDR-H3 and the CDR-L1 region of antibodies may significantly vary in length. Thus, insertions and deletions of one or more amino acids in, for example these regions compared to the CDR-L1 and CDH-H3 amino acid sequence described here, are also encompassed within the meaning of "variants". Thus, by the term "variants", as used in the present invention, is meant either any NCAM binding molecule in which the hypervariable regions CDR-L1, CDR-L2, CDR-L3, CDR-H1, CDR-H2, and CDR-H3 taken as a whole have at least 80% sequence identity, preferably at least 90% sequence identity, more preferably at least 95% sequence identity to the hypervariable regions as shown in SEQ ID Nos. 6, 8, 10, 12, 14, and 16, and, which is capable of binding to NCAM substantially at least to the same extent as a reference molecule having hypervariable regions CDR-L1, CDR-L2, CDR-L3, CDR-H1, CDR-H2, and CDR-H3 identical to those shown in SEQ ID Nos. 6, 8, 10, 12, 14, and 16. By the term "to the same extent" is meant that the reference and the equivalent molecules exhibit, on a statistical basis, essentially identical NCAM binding properties in one of the utilized bioassays referred to below. The term "sequence identity" as used in the present invention means the percentage of pair-wise identical residues-following homology alignment of a sequence of a polypeptide of the present invention with a sequence in question-with respect to the number of residues in the longer of these two sequences. Thus, in the context of the present invention a "variant" of an NCAM-binding molecule is a polypeptide which contains one or more modifications to the primary amino acid sequence while retaining the binding properties, e.g. in terms of affinity, disclosed herein. If a variant of an NCAM-binding molecule involves an amino acid substitution, conservative amino acid substitutions typically may be preferred, i.e., substitutions which retain a property of the original amino acid such as charge, hydrophobicity, conformation, etc. Examples of conservative substitutions of amino acids include, but are no limited to, substitutions made among amino acids within the following groups: (1) M, I, L, V; (2) F, Y, W; (3) K, R, H; (4) A, G; (5) S, T; (6) Q, N; and (7) E, D.

Modifications, which generate variants of NCAM-binding molecules, may be made in order to enhance protein stability in an expression system, to enhance the stability of protein-protein binding such as immunglobulin-NCAM binding, or to increase the avidity. Again, any method for preparing modified or variant peptides can be employed, such as synthesis of the modified or variant peptide or its recombinant production using a mutated nucleic acid molecule.

As used herein, "fragment" refers to any N-terminally and/or C-terminally shortened NCAM-binding molecule, which is capable to bind NCAM substantially to the same extent as the full-length molecule. In particular, "fragments" relates to immunoglobulins of the invention missing one or more of the N-terminal and/or C-terminal amino acids in which the NCAM-binding properties are substantially retained. In the context of antibodies, "fragment" can also refer to, for example, Fab, Fv, single chain Fv, and Fc antibody fragments, all of which are known to the skilled person.

In the context of the invention, "derivatives" refers to immunoglobulin proteins in which anyone or several amino acids have been altered, but which retain substantially identical properties. Said alteration techniques are well known to those skilled in the art and include modification of amino acids by adding or eliminating a substituent, and replacing natural L-amino acids with the D-enantiomers, any non-natural amino acids or any other suitable chemical compound. Non-naturally occurring amino acids include, but are not limited to, hydroxyprolin, aminoprolin, thyroxin, ornithine, norvaline, norleucine, beta-alanine, gamma-amino butyric acid, homoserine, citrulline and the like. Naturally occurring amino acids include glycine, alanine, valine, leucine, isoleucine, methionine, proline, phenylalanine, tyrosine, tryptophan, serine, threonine, cysteine, glutamine, asparagine, histidine, lysine, arginine, glutamic acid and aspartic acid. As mentioned above, enantiomers of above-listed amino acids, i.e. the L- and the respective D-form, are also included. In this context "derivatives" also refers to the above listed amino acid that are modified by additional functional groups, preferably hydroxy, carboxy, amino, C₁-C₆ alkyl, amido, and guanido, all of which are well known to those skilled in the art.

In a second aspect, the invention is directed to a monoclonal antibody, termed 5A2, obtainable from a hybridoma which secretes anti-NCAM antibody, wherein the hybridoma is identified by deposit number DSM ACC2757. Said antibody comprises a chimeric heavy chain, comprising the constant region of a human heavy chain and a murine variable domain, and a fully murine light chain. The hybridoma with the deposit number DSM ACC2757, which secretes the monoclonal anti-NCAM antibody of the invention, is also part of the invention. Also any fragment of the antibody 5A2, for example, an Fab or Fab' fragment that is either obtain by proteolysis of the intact antibody 5A2 (for example by papain) or recombinantly produced is also encompassed in the present invention. Other illustrative examples of recombinantly produced fragments that are encompassed herein are scFv fragments.

Both said monoclonal antibody and the above immunoglobulin molecule can be used as a medicament.

The above-described immunoglobulin molecules or antibodies according to the invention may have an affinity (dissociation constant K_{D}) for NCAM of at least 1 x 10⁻⁶ M⁻¹, preferably 1 x 10⁻⁷ M⁻¹ or more. As further detailed below in the Examples section, the dissociation constant K_{D} of the antibodies may be determined by ¹³¹I-labeling of the antibodies, usage of NCAM expressing IMR5-75 neuroblastoma cells as binding partners, measuring cell specific radioactivity, and using the scatchard plot method to determine the K_{D} of radio-iodinated antibodies.

### Preparation

An NCAM-binding molecule of the invention may be produced by recombinant DNA techniques. In view of this, one or more DNA molecules encoding the binding molecule, preferably one or more immunoglobulin molecules, must be constructed, placed under appropriate control sequences and transferred into a suitable host organism for expression.

In a very general manner, there are accordingly provided (i) DNA molecules encoding a single domain NCAM-binding molecule of the invention, a single chain NCAM-binding molecule of the invention, a heavy or light chain or fragments thereof of a NCAM-binding molecule of the invention and (ii) the use of the DNA molecules of the invention for the production of a NCAM-binding molecule of the invention by recombinant means.

The present state of the art is such that the skilled man will be able to synthesize the DNA molecules of the invention given the information provided herein i.e. the amino acid sequences of the hypervariable regions and the DNA sequences coding for them.

Furthermore, it is not necessary to have access to the mRNA from a producing hybridoma cell line in order to obtain a DNA construct coding for the MAbs of the invention. Thus, PCT application W0 90/07861 gives full instructions for the production of a MAb by recombinant DNA techniques given only written information as to the nucleotide sequence of the gene. The method comprises the synthesis of a number of oligonucleotides, their amplification by the PCR method, and their splicing to give the desired DNA sequence.

Expression vectors comprising a suitable promoter or genes encoding heavy and light chain constant parts are publicly available. Thus, once a DNA molecule of the invention is prepared it may be conveniently transferred in an appropriate expression vector. DNA molecules encoding single chain antibodies may also be prepared by standard methods, for example, as described in W0 88/1649.

The DNA constructs of the invention, for example encoding an immunoglobulin heavy and a immunoglobulin light chain, may be placed under the control of suitable control sequences, in particular under the control of a suitable promoter. Any kind of promoter may be used, provided that it is adapted to the host organism in which the DNA constructs will be transferred for expression. However, if expression is to take place in a mammalian cell, it is particularly preferred to use the promoter of an immunoglobulin gene.

The desired antibody may be produced in a cell culture or in a transgenic animal. A suitable transgenic animal may be obtained according to standard methods.

When the antibody chains have to be produced in a cell culture, the DNA constructs must first be inserted into either a single expression vector or into two separate but compatible expression vectors, the latter possibility being preferred.

### Conjugates

Conjugates of the NCAM-binding molecules of the invention, e.g. toxin or radioisotope conjugates, are also included within the scope of the invention.

Although naked anti-NCAM/anti-CD56 immunoglobulin molecules/antibodies are the primary object of the present invention, therapeutically useful immunoconjugates in which an immunoglobulin component is conjugated to a radionuclide, a toxin or a chemotherapeutic drug are also encompassed by the present invention.

In one embodiment of the invention the immunoglobulin molecules may be radiolabelled, with the radiolabel preferably selected from the group consisting of ³²P, ⁶⁷Cu, ⁹⁰Y, ¹¹¹In, ¹²⁵I, ¹³¹I, ¹⁸⁶Re, ¹⁸⁸Re, ¹⁹⁸Au, and ²¹¹At.

A radioisotope can be attached to an antibody component directly or indirectly, via a chelating agent. For example, ⁶⁷Cu, considered one of the more promising radioisotopes for radioimmunotherapy due to its 61.5 hour half-life and abundant supply of beta particles and gamma rays, can be conjugated to an antibody component using the chelating agent, p-bromoacetamido-benzyl-tetraethylamine-tetraacetic acid (TETA) (Chase, "Medical Applications of Radioisotopes," in REMINGTON'S PHARMACEUTICAL SCIENCES, 18th Edition, Gennaro et al. (eds.), pages 624-652 (Mack Publishing Co. 1990)).

In a further embodiment, the invention features immunoglobulin molecules according to the invention which are conjugated to a toxin, with the toxin being preferably selected from the group consisting of maytansine, N^{2'}-deacetyl-N^{2'}-(3-mercapto-1-oxopropyl)-maytansine, ricin, abrin, ribonuclease, DNase I, Staphylococcal enterotoxin-A, pokeweed antiviral protein, gelonin, diphtherin toxin, *Vinca* alkaloids, *Pseudomonas* endo- and exotoxin or derivatives and pharmaceutically acceptable salts thereof (Pastan et al. Cell 47:641, 1986; Goldenberg - A Cancer Journal for Clinicians 44:43, 1994). Other suitable toxins are known to those of skill in the art.

An alternative approach to introducing the combination of immunoglobulin and toxin is provided by immunoglobulin-toxin fusion proteins. An immunoglobulin-toxin fusion protein is a fusion protein that comprises an antibody moiety and a toxin moiety. Methods for making immunoglobulin-toxin fusion proteins are known to those of skill in the art, for example from Chaudhary et al., Nature 339:394, 1989; Brinkmann et al., Proc. Natl. Acad. Sci. USA 88:8616, 1991; Batra et al., Proc. Natl. Acad. Sci. USA 89:5867, 1992; Friedman et al., J. Immunol. 150:3054, 1993 ; Wels et al., Int. J. Can. 60:137, 1995; Fominaya et al., J. Biol, Chem. 271:10560, 1996; Kuan et al., Biochemistry 35:2872, 1996; Schmidt et al., Int. J. Can. 65:538, 1996 ; Kreitman et al., Leukemia 7:553, 1993; Nicholls et al., J. Biol. Chem. 268:5302, 1993 ; Thompson et al., J. Biol. Chem. 270:28037, 1995; Vallera et al., Blood 88:2342, 1996; Deonarain et al., Tumor Targeting 1:177, 1995; Linardou et al., Cell Biophys. 24-25:243, 1994; Wang et al., Abstracts of the 209th ACS National Meeting, Anaheim, CA, 2-6 April, 1995, Part 1., BIOTOOS; and Dohlsten et al., Proc. Natl. Acad. Sci. USA 91:8945, 1994.

Useful cancer chemotherapeutic drugs for the preparation of immunoconjugates include nitrogen mustards, alkyl sulfonates, nitrosoureas, triazenes, folic acid analogs, pyrimidine analogs, purine analogs, antibiotics, epipodophyllotoxins, platinum coordination complexes, hormones, and the like. Suitable chemotherapeutic agents are described in REMINGTON'S PHARMACEUTICAL SCIENCES, 19th Ed. (Mack Publishing Co. 1995), and in GOODMAN AND GILMAN'S THE PHARMACOLOGICAL BASIS OF THERAPEUTICS, 7th Ed. (MacMillan Publishing Co. 1985). Other suitable chemotherapeutic agents, such as experimental drugs, are known to those of skill in the art.

Such immunoconjugates and immunoglobulin-immunomodulator fusion proteins also provide a means to deliver an immunomodulator to a target cell and are particularly useful against tumor cells. The cytotoxic effects of immunomodulators are well known to those of skill in the art. See, for example, Klegerman et al., "Lymphokines and Monokines," in BIOTECHNOLOGY AND PHARMACY, Pessuto et al. (eds.), pages 53-70 (Chapman & Hall 1993). As an illustration, interferons can inhibit cell proliferation by inducing increased expression of class I histocompatibility antigens on the surface of various cells and thus, enhance the rate of destruction of cells by cytotoxic T lymphocytes. Furthermore, tumor necrosis factors, such as TNF-α, are believed to produce cytotoxic effects by inducing DNA fragmentation. Thus, the immunoglobulins of the present invention can comprise an immunomodulator moiety.

As used herein, the term "immunomodulator" includes cytokines, stem cell growth factors, lymphotoxins, such as tumor necrosis factor (TNF), and hematopoietic factors, such as interleukins (e.g., interleukin-1 (IL-1), IL-2, IL-3, IL6, IL-10 and IL-12), colony stimulating factors (e.g., granulocyte-colony stimulating factor (G-CSF) and granulocyte macrophage-colony stimulating factor (GM CSF)), interferons (e.g., interferons-α, -β and -γ), the stem cell growth factor designated "S1 factor," erythropoietin and thrombopoietin. Examples of suitable immunomodulator moieties include IL-2, IL-6, IL-10, IL12, interferon-, TNF-α, and the like.

As a further illustration, a therapeutic agent can be attached at the hinge region of a reduced antibody component via disulfide bond formation. For example, the tetanus toxoid peptides can be constructed with a single cysteine residue that is used to attach the peptide to an antibody component. As an alternative, such peptides can be attached to the antibody component using a heterobifunctional cross-linker, such as N-succinyl 3-(2pyridyldithio)proprionate (SPDP) (Yu et al., Int. J. Cancer 56:244, 1994). General techniques for such conjugation are well-known in the art (See, for example, Wong, CHEMISTRY OF PROTEIN CONJUGATION AND CROSS-LINKING (CRC Press 1991); Upeslacis et al., "Modification of Antibodies by Chemical Methods," in MONOCLONAL ANTIBODIES: PRINCIPLES AND APPLICATIONS, Birch et al. (eds.), pages 187-230 (Wiley-Liss, Inc. 1995); Price, "Production and Characterization of Synthetic Peptide Derived Antibodies," in MONOCLONAL ANTIBODIES: PRODUCTION, ENGINEERING AND CLINICAL APPLICATION, Ritter et al. (eds.), pages 60-84 (Cambridge University Press 1995).

As described above, carbohydrate moieties in the Fc region of an antibody can be used to conjugate a therapeutic agent. However, the Fc region is absent if an antibody fragment is used as the antibody component of the immunoconjugate. Nevertheless, it is possible to introduce a carbohydrate moiety into the light chain variable region of an antibody or antibody fragment. The engineered carbohydrate moiety is then used to attach a therapeutic agent. Moreover, the carbohydrate moiety can also be used to attach polyethyleneglycol in order to extend the halflife of an intact antibody, or antigen-binding fragment thereof, in blood, lymph, or other extracellular fluids.

In addition, those of skill in the art will recognize numerous possible variations of the conjugation methods and will be able to devise conjugation schemes without undue experimentation.

### Compositions

In a third aspect, the present invention relates to a pharmaceutical composition comprising an immunoglobulin molecule or antibody according to the invention and optionally a pharmaceutically acceptable carrier. Additionally, the compositions of the invention can further comprise an excipient, auxiliary, preservative, buffer agent, solvent or the like, all of which are known to a person skilled in the pharmaceutical art. In such compositions, the immunoglobulin molecules or antibodies of the invention may be in lyophilized form.

The compositions containing an NCAM binding molecule, immunoglobulin, or antibody can be used as medicament or for the manufacture of a pharmaceutical for the diagnosis, treatment and/or prevention of NCAM-related diseases.

"NCAM-related diseases" are such diseases that are characterized by increased NCAM expression. As high NCAM-expression is especially linked to certain malignancies and autoimmune disorders, also referred to as "NCAM-related malignancies" and "NCAM-related autoimmune disorders", the present invention is particularly directed to the diagnosis, treatment and/or prevention of these. To be recognized by the immunoglobulin molecules of the present invention, the aberrant cells have to present NCAM on the cell surface. Examples for NCAM-related diseases will be discussed below in more detail.

Pharmaceutical compositions of the invention may be manufactured in conventional manner. A composition according to the invention may be provided in lyophilized form. For immediate administration it is dissolved in a suitable aqueous carrier, for example sterile water for injection or sterile buffered physiological saline. If it is considered desirable to make up a solution of larger volume for administration by infusion rather than as a bolus injection, it is advantageous to incorporate human serum albumin or the patient's own heparinized blood into the saline at the time of formulation. The presence of an excess of such physiologically inert protein prevents loss of monoclonal antibody by adsorption onto the walls of the container and tubing used with the infusion solution. If albumin is used, a suitable concentration is from 0.5 to 4.5% by weight of the saline solution.

### Utilization

In a further aspect, the present invention is directed to the use of the compositions of the invention for the manufacture of a pharmaceutical for the treatment or prevention of NCAM-related diseases in a subject, with the subject being preferably a mammal, more preferably a human.

The above use for the treatment of NCAM-related malignancies and autoimmune disorders can further comprise administering to a patient a therapeutically effective amount of an NCAM-binding molecule according to the invention. Said NCAM-binding molecule, is preferably an immunoglobulin, more preferably an antibody, most preferably a chimeric or humanized anti-NCAM antibody according to the invention.

A composition or antibody according to the invention is said to be administered in a "therapeutically effective amount" if the amount administered is physiologically significant. An agent is physiologically significant if its presence results in a detectable change in the physiology of a recipient patient. In the present context, an agent is physiologically significant if its presence results in the inhibition of the growth of target tumor cells, or prevents, blocks, or inhibits an autoimmune reaction.

The efficacy of such anti-NCAM/anti-CD56 antibody treatment can, dependent on the intended use, be enhanced by supplementing naked antibodies with different kinds of immunomodulators such as cytokines, growth factors, other antibodies and antibody derivatives. In such multimodal regimens, the supplemental therapeutic compositions can be administered before, concurrently or after administration of naked anti-NCAM immunoglobulins/antibodies.

The treatment of NCAM-related malignancies with a therapeutically effective amount of an NCAM-binding molecule can be applied before, during or subsequent to a chemotherapeutic regimen. Such a chemotherapy regimen may be selected from the group consisting of, at the very least, CHOP, ICE, Mitoxantrone, Cytarabine, DVP, ATRA, Idarubicin, hoelzer chemotherapy regime, La La chemotherapy regime, ABVD, CEOP, 2-CdA, FLAG & IDA with or without subsequent G-CSF treatment, VAD, M & P, C-Weekly, ABCM, MOPP, DHAP and many other mono- or polychemotherapeutic regimens. Therefore, if the compositions of the invention are used for treating malignancies, they can be combined with one or more chemotherapeutic agent(s), for example selected from the group consisting of cyclophosphamide, etoposide, vincristine, procarbazine, prednisone, carmustine, doxorubicin, methotrexate, bleomycin, dexamethasone, phenyl butyrate, bryostatin-1 and leucovorin.

Said treatment of NCAM-related autoimmune disorders with a therapeutically effective amount of an NCAM-binding molecule can be applied before, during or subsequent to a immunosuppressive or immunomodulatory regimen. Such a regimen may be selected from the group including Azathioprine, Methotrexate, Tacrolimus and derivates, Montelukast, Glucocorticoides, Cyclophosphamide, Cyclosporine, Leflunomide, anti-TNF therapy (e.g. antibodies), antibodies against lymphocytes and others.

The treatment of NCAM-related malignancies with a therapeutically effective amount of an NCAM-binding molecule can also be applied before, during or subsequent to radiotherapy.

The combined therapies of the present invention also include the use of the compositions or antibodies of the invention for the manufacture of a pharmaceutical for the treatment of NCAM-related malignancies and autoimmune disorders comprising administering at least one chimeric anti-NCAM immunoglobulin/antibody and at least one cytokine. Preferred cytokines are selected from the group consisting of alpha interferon, gamma interferon, IL-2, IL-12, GM-CSF and G-CSF.

Again, the anti-NCAM immunoglobulin/antibody and the cytokine(s) may be administered sequentially, in either order, or in combination.

It should be clear that the combined therapeutic regimens of the present invention can be performed whereby said therapies are given simultaneously, i.e., the anti-NCAM immunoglobulin/antibody is administered concurrently or within the same time frame (i.e., the therapies are going on concurrently, but the agents are not administered precisely at the same time). The anti-NCAM immunoglobulins/antibodies of the present invention may also be administered prior to or subsequent to the other therapies. Sequential administration may be performed regardless of whether the patient responds to the first therapy to decrease the possibility of remission or relapse.

The NCAM-related malignancies to be treated according to the present invention may be of neuroendocrine origin. In specific embodiments, the NCAM-related malignancies include, but are not limited to, neuroblastoma, brain tumor, rhabdomyosarcoma, small cell lung cancer, nephroblastoma (Wilms' tumor), carcinoid, pituitary tumor, acute non-lymphocytic leukemia (ANLL), acute monocytic leukemia (AMoL), acute megakaryocytic leukemia (AMKL), multiple myeloma, myeloid leukemia and large granular lymphocytic leukemia. In a preferred embodiment, said malignancy is a neuroblastoma.

The preferred autoimmune disorder to be treated according to the invention is rheumatoid arthritis.

In another aspect the compositions according to the invention are used for diagnostic purposes, i.e. for the detection or diagnosis of NCAM-related diseases.

### Dosage/administration

Additional pharmaceutical methods may be employed to control the duration of action of an immunoglobulin component, immunoconjugate or fusion protein in a therapeutic application. Control release preparations can be prepared through the use of polymers to complex or adsorb the antibody component, immunoconjugate or fusion protein. For example, biocompatible polymers include matrices of poly(ethylene-co-vinyl acetate) and matrices of a polyanhydride copolymer of a stearic acid dimer and sebacic acid. The rate of release of an antibody component (or immunoconjugate) from such a matrix depends upon the molecular weight of the protein, the amount of antibody component/ immunoconjugate/ fusion protein within the matrix, and the size of dispersed particles (Saltzman et al., Biophys. J. 55:163, 1989; Sherwood et al., Bio/Technology 10:1446, 1992). Other solid dosage forms are described in Remington's Pharmaceutical Sciences, 19th ed. (1995).

The NCAM-binding molecules, anti-NCAM immunoglobulins and antibodies as well as fragments, variants and derivatives thereof of the present invention can be formulated according to known methods to prepare pharmaceutically useful compositions, whereby the therapeutic proteins are combined in a mixture with a pharmaceutically acceptable carrier.

In this context, "pharmaceutically acceptable" refers to the fact that the administration of a certain compound, carrier, excipient and the like, is well-tolerated by the recipient.

Sterile phosphate-buffered saline is one example of a pharmaceutically acceptable carrier. Other suitable carriers are well-known to those in the art.

In general, the dosage of administered NCAM-binding molecules, anti-NCAM immunoglobulins and antibodies as well as fragments, variants and derivatives, immunoconjugates, and fusion proteins thereof will vary depending upon such factors as the patient's age, weight, height, sex, general medical condition and previous medical history. Typically, it is desirable to provide the recipient with a dosage in the range of from about 1 µg/kg to 10 mg/kg (amount of agent/body weight of patient), although a lower or higher dosage also may be administered as circumstances dictate.

Administration of the immunoglobulins/antibodies of the present invention to a patient can be intravenous, intraperitoneal, intramuscular, subcutaneous, intrapleural, intrathecal, by perfusion through a regional catheter, or by direct intralesional injection. Of these routes, intravenous administration is most preferred. When administering therapeutic proteins by injection, the administration may be by continuous infusion or by single or multiple boluses.

Those of skill in the art are aware that intravenous injection provides a useful mode of administration due to the thoroughness of the circulation in rapidly distributing antibodies. Intravenous administration, however, is subject to limitation by a vascular barrier comprising endothelial cells of the vasculature and the subendothelial matrix.

Preferably, naked anti-NCAM antibodies are administered at low protein doses, such as 20 to 100 milligrams protein per dose, given once, or repeatedly, parenterally.

The present invention, thus generally described, will be understood more readily by reference to the following examples, which are provided by way of illustration and are not intended to be limiting of the present invention.

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention, which is defined by the following claims.

Unless otherwise indicated, any polypeptide chain is herein described as having an amino acid sequence starting at the N-terminus and ending at the C-terminus.

### Examples

Illustrative antibodies according to the present invention were generated by using mice that harbor human immunoglobulin fragments instead of their murine counterparts. Two mouse strains were used for antibody engineering: F004 mice were used for generating the ch.MK1 monoclonal antibody and F004-Jen mice were used for generating the 5A2 monoclonal Ab.

### Generation of the new F004-Jen mouse strain

F004 Mice (Zou et al., Curr. Biol. 4:1099-1103, 1994; Zou et al., Science 262:1271-1274, 1993) were obtained from Miltenyi Biotech Inc. (Bergisch Gladbach, Germany). Embryo transfer and testing for murine pathogens were performed by Charles River Laboratories (St.-Germain sur l'Arbresle, France). C57B1/6 mice were purchased from Taconic M&B (Bomholt, Denmark). Male and female mice were generally paired at a 1:1 ratio. All animal experiments were carried out in accordance with institutional and national guidelines.

Since breeding results of F004 mice were poor, modification of the genetic background by crossing F004 mice with the more fertile C57Bl/6 mice was necessary to enhance breeding results and preserve the important immunoglobulin replacement mutations for future work.

F004 mice were mated with C57Bl/6 mice to obtain a F1 daughter generation. All F1 mice heterozygously expressed both mutations. Mice from the F1 generation were subsequently backcrossed to F004 mice. This step was performed to obtain a next daughter generation (F2) were both mutations were present at enhanced frequencies as compared with F1 mice. Mice from the F2 generation that were homozygous for both mutations were identified by ELISA screening and used as founders for the novel F004-Jen strain. The new F004-Jen mice were subsequently selected for gray coat color to distinguish the agouti F004 from F004-Jen mice. Additionally all other (non-F004-Jen) F2 mice were also bred for one more generation to analyze their reproduction capacity as well for comparison reasons.

The new F004-Jen strain demonstrated significantly improved breeding results as compared with the F004 strain and were used for generating anti-NCAM antibodies.

### Analysis of human Cγ1 and Cκ expression levels in F004 and F004-Jen mice

For determining human IgG1 or alternatively murine IgG1 (for comparison) serum concentrations microtiter plates were coated with goat anti-human-IgG or anti-mouse-IgG1 Abs. Plates were blocked with 20% FCS. Serum samples were diluted stepwise (1:20 up to 1:40,960) and incubated on the 96 well plastic plates as well as reference antibodies (e.g. ch.KLH) at predefined concentrations. Abs were detected by goat anti-human-IgG-HRPO (horseradish peroxidase) or goat anti-mouse-IgGl-HRPO, respectively.

To assess light chain expression on the surface of B cells, freshly isolated splenocytes from non-immunized 6-12 week old mice were stained with anti-mouse-CD19-PE to identify B cells and goat anti-human-κ, anti-mouse-λ-, anti-mouse κ-, anti-human IgG- or anti-mouse IgG1-FITC Abs (all Abs Southern Biotechnology). Dead cells were excluded by 7-AAD staining. Cells were analyzed using a FACSCalibur flow cytometer.

Human Cγ1 usage in hybridoma cell lines was determined from primary hybridoma cultures. Human IgG1-, murine IgG2a, -2b or -3 production was determined 10 days after cell fusion by analyzing supernatants from primary cell cultures (96 well plates): 96 well ELISA plates were coated with capture Abs against the respective Ig isotypes and plates blocked with 20% FCS. Supernatants were incubated and captured Abs were detected by secondary HRPO coupled Abs directed against the respective isotype (1 µg/ml, all Southern Biotechnology). Cut off was twofold over background before a test was considered as positive. All results demonstrated equal or nearly equal phenotype and protein expression levels of F004 and F004-Jen mice regarding both (γ1 and κ) replacement mutations. The results indicate the new F004-Jen strain being similar effective for chimeric antibody generation as the F004 strain.

### Tumor cell lines and general cell culture

Tumor cells were cultured in RPMI1640 medium (PAA AG, Linz, Austria), supplemented with 10% heat inactivated fetal calf serum (Biochrome, Berlin, Germany) and 10 mg/ml ciprofloxacin (Bayer, Leverkusen, Germany), referred to here as standard medium (SM). Hybridoma cells were cultured in SM or alternatively in serum free hybridoma express medium (PAA AG), supplemented with 2 mM L-glutamine (PAA AG) and ciprofloxacin for serum free antibody production. Tumor cell lines used: (1) neuroblastoma IMR-32 (ATCC: CCL-127), IMR5 (Jensen et al., Clin. Exp. Immunol. 134:253-263, 2003), and IMR5-75 (Jensen et al., Immunol. Lett. 93:205-210, 2004); (2) small cell lung cancer H69 (ATCC: HTB-119); (3) colorectal cancer H498 (ATCC: CCL-254) and (4) natural killer cell leukemia NK-92 (DSMZ: ACC488). NK-92 cells were grown in α-MEM medium (Gibco) supplemented with 12.5 % FCS, 12.5 % horse serum (PAA AG), 2 mM L-glutamine and 50 U/ml human Interleukin-2 (Chiron-Behring, Marburg, Germany). P3X63Ag8 murine myeloma cells (ATCC: TIB-9) used as a fusion partner for hybridoma production were cultured in SM.

### Antibodies

Antibodies were purified from serum free hybridoma supernatants by protein G affinity chromatography (Hombach et al., J Immunother. 20:325-333, 1997). Chimeric ch14.18 Abs specifically recognize the GD2 ganglioside. Clinical grade material of the ch14.1.8 Ab was a gift from the German Neuroblastoma Study Group. Chimeric Trastuzumab Abs bind the Her2/neu antigen. Clinically grade material was obtained from Roche (Grenzach, Swizerland).

### Antigens used for antibody screening

PSA-NCAM, i.e. NCAM molecules that carry polysialic acid molecules on their surface modifying their immunogenicity, was purified from Kgla and TE671 cell lysate by PSA specific affinity chromatography, using immobilized Ab NmB735. In human organisms NCAM is preferentially found in its polysialated form, which was reason to use PSA-NCAM for the present work. Purified GD2 gangliosides were purchased (Calbiochem, Bad Soden, Germany). NCAM peptide sequences were selected from the third Ig-like domain, which is an antigen dominant region (Gerardy-Schahn et al., Int. J. Cancer Suppl. 8:38-42, 1994), by querying NCAM140 protein sequence to the BCEPred server (Saha et al. Eds, ICARIS 2004, LNCS 3239. Berlin, Germany: Springer: 197-204, 2004). Finally NCAM₂₅₅-₂₇₀(WITA, Teltow, Germany) and NCAM₂₇₂₋₂₈₅, ₂₈₉₋₃₀₄ (Perbio, Bonn, Germany) peptides were synthesized.

### Mouse immunization and hybridoma fusion

F004 mice or F004-Jen mice were immunized for 31 days (Peters et al., Monoklonale Antikörper. 1th ed. Heidelberg, Germany: Springer; 1990) with weekly i.p. injections of Keyhole limpet hemocyanine (KLH) to generate Abs against KLH or alternatively with 2 x 10⁶ irradiated IMR5 neuroblastoma cells to generate anti-KLH or anti-NCAM Abs respectively. On days -3, -2 and -1 before spleens were removed, mice were additionally boostered i.v. with KLH or 1 x 10⁶ irradiated IMR5 neuroblastoma cells, respectively.

To get a more robust B cell response against NCAM, another group of mice were immunized with keyhole limpet hemocyanine (EDC-KLH coupling kit, Pierce, Bonn, Germany) coupled NCAM₂₇₂-₂₈₅ peptides plus GerbuMM adjuvant (Gerbu Biochemicals, Gaiburg, Germany) followed by two bi-weekly i.p. injections of (i) 2 x 10⁷ irradiated human NCAM expressing NK-cells and (ii) 2 x 10⁷ IMR32 neuroblastoma cells before mice were boostered i.v. with IMR32 cells and spleens removed.

Hybridoma fusion was performed using a standard protocol (Peters et al., Monoclonal Antibodies; 1991) and ^{HGPRT-}P3X63Ag8 cells as fusion partner. Hybridoma clones growing in SM supplemented with HAT (2.5 x 10⁻³ M hypoxanthine, 1 x 10⁻⁵ aminopterin, 4 x 10⁻⁴ M thymidine, Sigma, Deisenhofen, Germany) for cell selection and 50 U/ml murine IL-6 (Roche Molecular Biochemicals, Mannheim, Germany) and were screened by antigen specific ELISA, using a direct ELISA with immobilized KLH or PSA-NCAM respectively.

PSA-NCAM was used as a screening antigen to generate chimeric Abs, which can bind to NCAM in its polysialylated form. Alternatively, NCAM peptides (NCAM₂₅₅₋₂₇₀, ₂₇₂₋₂₈₅, ₂₈₉₋₃₀₄) were used for screening to identify Abs that can specifically bind the third Ig-like NCAM domain. To immobilize antigens on microtiterplates NCAM peptides (0.5 µg/ml) or KLH (0.1 µg/ml) was diluted in PBS and 50 µl/well were dried on a microtiter plate at 60ºC for 3 hours. PSA-NCAM was diluted in PBS (1 µg/ml) and incubated on ELISA plates for 60 minutes. Plates with immobilized antigens were washed and blocked with 20% FCS in PBS. Approximately 100 µl hybridoma cell culture supernatant from each well was then transferred to the ELISA plates to allow binding of the chimeric Abs to immobilized antigens. Chimeric Abs were detected with goat anti-human IgG-HRPO (horseradish peroxidase) (1 µg/ml) or alternatively with goat anti-human Ig(H+L) biotin plus streptavidin-HRPO (both 0.5 µg/ml; all reagents from Southern Biotechnology, Malvern, PA). O-phenylenediamine-dihydrochloride (OPD, Sigma) was used as a substrate and reaction was stopped by adding 50 µl/well 1.3 M H₂SO₄ per well. Clones identified were subcloned by limiting dilution in SM supplemented with 50 U/ml murine IL-6 to stimulate cell growth.

The ch.MK1 antibody was developed by screening with immobilized PSA-NCAM, the 5A2 antibody was developed by screening with NCAM peptides (NCAM₂₅₅₋₂₇₀, ₂₇₂₋₂₈₅, ₂₈₉₋₃₀₄) and the ch.KLH antibody was developed by screening with KLH.

### Determination of the molecular weight of the new Abs

For determining the molecular weight of new chimeric antibodies a non-reducing capillary SDS-PAGE (Bioanalyzer^{™}, Agilent, Waldbronn, Germany) was employed following the manufacturers instructions. Purified ch.MK1 Abs have a molecular weight of approximately 170 KD and ch.KLH Abs of approximately 155 KD.

### Assessment of antibody binding to NCAM expressing cells

Ch.MK1 binding to cell surfaces of viable cells was tested by flow cytometry: H69, IMR32, NK-92 and H498 tumor cells were incubated with ch.MK1 Abs or (5 µg/ml) for 15 minutes at 4ºC. Cells were washed and Abs detected by goat anti-human IgG-biotin and streptavidin-FITC (Southern Biotechnology). Cells were measured in a FACSCalibur or alternatively FACSCanto flow cytometer (Becton Dickinson). Dead cells were excluded by 7-AAD (Sigma) staining.

### Assessment of the dissociation constant of ch.MK1 antibodies

The dissociation constant K_{D} of the ch.MK1 antibody was measured using ¹³¹I-labeling and NCAM expressing IMR5-75 neuroblastoma cells as binding partner. ¹³¹I-labeling was carried out by the chloramin T method (Greenwood et al., Biochem. J. 89:114-123, 1963). Portions of 1 x 10⁵ cells in isotonic saline were incubated at room temperature with different concentrations of radio-labeled ch.MK1 Abs, in presence or absence of non-radioactive ch.MK1 (double determinations). After 30 min incubation cells were centrifuged and supernatants were removed. Then cells were washed with isotonic saline, centrifuged, and supernatants removed again. Cell specific radioactivity was measured using a well counter (MED, Dresden, Germany). The scatchard plot method was used to determine the K_{D} of radio-iodinated ch.MK1 (Scatchard G, Ann. NY Acad. Sci. 51;660-672, 1949). The dissociation constant K_{D} of the ch.MK1 antibody was determined as 4.3-8.7 x 10⁻⁸ M. The dissociation constant K_{D} of the 5A2 antibody was determined in the same way as 1.0-2.0 x 10⁻⁸ M.

### Cytotoxic function of ch.MK1 on NCAM+ cells

Functional activity of ch.MK1 against NCAM expressing cells was investigated in a flow cytometry based assay, using freshly isolated PBMC as a responder population and NCAM expressing NK cells derived from peripheral blood. PBMC were incubated with 2 µg/ml of the respective mAbs (ch.MK1, ERIC1, ch14.18) or medium alone for 6-7 days. In an additional series of experiments, IL2 was added at 100 U/ml. Cells were then stained with CD16-FITC (Becton Dickinson) to detect remaining CD16+ NK cells and 7-AAD to identify dead cells. The number of viable CD16+ cells was subsequently assessed by flow cytometry.

### SCID mouse experiments

To test the inhibitory effect of anti-NCAM Abs on IMR-5/75 tumour growth, a xenograft model was established in SCID mice. SCID mice were subcutaneously challenged with 2 x 10⁷ NCAM+ human neuroblastoma cells (IMR-5/75) to induce subcutaneous growth of a tumour nodule. Treatment started after visible tumour growth was detected in all mice.
(1) Mice were treated at day 9, 10, 13, 14, 16 and 17 with subcutaneous injection of 2 x 10⁷ freshly isolated human PBMC plus 200 µg ch.MK1 Abs or PBMC alone or PBS only (phosphate buffered saline) as a control (figure 6A); or
(2) Mice were treated intravenously at day 14 with 2 x 10⁷ freshly isolated human PBMC plus 100 µg 5A2 Abs (closed circles) or PBMC alone (open circles) as a control. Treatment was intensified on day 19, 21, and 24 by injecting 2 x 10⁷ freshly isolated human PBMC plus 100 µg 5A2 Abs intravenously and an additional intralesional injection of the same doses (figure 6B).

Results for ch.MK1 (1): Endpoint was 1000 mm³ tumor volume. Box plots (figure 6A) show median value and range of each group. A significant therapeutic effect for the ch.MK1 antibody was demonstrated. P = 0.0054 (logrank test).

Results for 5A2 (2): At day 25 and 26 tumor sizes differed significantly between experimental and control group using the students t-test to compare tumor sizes at each time point, demonstrating a significant therapeutic effect of the 5A2 antibodies.

These experiments were carried out blinded with the veterinarian injecting mice and measuring tumor sizes not knowing which was the experimental group.

## Claims

1. An immunoglobulin molecule, which comprises at least
a) one immunoglobulin heavy chain or fragment thereof which comprises a variable domain comprising in sequence the hypervariable regions CDR-H1, CDR-H2, and CDR-H3, wherein said CDR-H1 has the amino acid sequence set forth in SEQ ID NO:12, said CDR-H2 has the amino acid sequence set forth in SEQ ID NO:14, and said CDR-H3 has the amino acid sequence set forth in SEQ ID NO:16; and
b) one immunoglobulin light chain or fragment thereof which comprises a variable domain comprising in sequence the hypervariable regions CDR-L1, CDR-L2, and CDR-L3, wherein said CDR-L1 has the amino acid sequence set forth in SEQ ID NO:6, said CDR-L2 has the amino acid sequence set forth in SEQ ID NO:8, and said CDR-L3 has the amino acid sequence set forth in SEQ ID NO:10;
or fragments, variants and derivatives thereof.

2. The immunoglobulin molecule according to claim 1, wherein said variable domain of an immunoglobulin heavy chain and said variable domain of an immunoglobulin light chain are covalently linked with each other by a peptide linker.

3. The immunoglobulin molecule according to claim 2, wherein said peptide linker consists of from about 4 to about 30 amino acids.

4. The immunoglobulin molecule according to claim 1, wherein said at least one immunoglobulin heavy chain further comprises the constant domain or a fragment thereof of a human heavy chain and wherein said at least one immunoglobulin light chain further comprises the constant part of fragment thereof of a human or murine light chain.

5. The immunoglobulin molecule according to claim 4, wherein
(i) the at least one heavy chain comprises a variable domain comprising the amino acid sequence shown in SEQ ID NO:2 and the constant domain of a human heavy chain, and
(ii) the at least one light chain comprises a variable domain having the amino acid sequence shown in SEQ ID NO:4 and the constant domain of a human or murine light chain,
or fragments, variants and derivatives thereof.

6. The immunoglobulin molecule according to claim 4 or 5, wherein the constant domain or fragment thereof of the human heavy chain is of the γ1 type and the constant domain or fragment thereof of the light chain is of the human κ type.

7. An immunoglobulin molecule according to any one of claims 4-6, comprising two light chains and two heavy chains.

8. The immunoglobulin molecule of claim 7, wherein said two light chains are identical to each other and wherein said two heavy chains are identical to each other.

9. An immunoglobulin molecule according to any one of claims 4-8, wherein said immunoglobulin molecule is a chimeric immunoglobulin molecule.

10. An immunoglobulin molecule according to any one of claims 4-8, wherein said immunoglobulin molecule is a humanized immunoglobulin molecule.

11. The immunoglobulin molecule of claim 9, wherein the variable domains are of murine origin.

12. The immunoglobulin molecule of claim 10, wherein the complementarity determining regions (CDRs) are of murine origin.

13. The immunoglobulin molecule of claim 12, wherein part of the framework region is of murine origin.

14. The immunoglobulin molecule according to any one of claims 1-13, wherein the affinity for NCAM expressed as the dissociation constant K_{D} is at least 1 x 10⁻⁶ M⁻¹.

15. A monoclonal antibody or a fragment, variant and derivative thereof, wherein said antibody is obtainable from a hybridoma which secretes said anti-NCAM antibody, said hybridoma being identified by deposit number DSM ACC2757.

16. The immunoglobulin molecule according to any one of claims 1-14 or the monoclonal antibody according to claim 15, wherein the immunoglobulin molecule or monoclonal antibody is radiolabeled.

17. The immunoglobulin molecule or monoclonal antibody according to claim 16, wherein the radiolabel is selected from ³²P, ⁶⁷Cu, ⁹⁰Y, ¹¹¹In, ¹²⁵I, ¹³¹I ¹⁸⁶Re, ¹⁸⁸Re, ¹⁹⁸Au, and ²¹¹At.

18. The immunoglobulin molecule according to any one of claims 1-14 or the monoclonal antibody according to claim 15, wherein the immunoglobulin molecule or monoclonal antibody is conjugated to a toxin.

19. The immunoglobulin molecule or monoclonal antibody according to claim 18, wherein the toxin is selected from maytansine, N^{2'}-deacetyl-N^{2'}-(3-mercapto-1-oxopropyl)-maytansine, ricin, abrin, ribonuclease, DNase I, Staphylococcal enterotoxin-A, pokeweed antiviral protein, gelonin, diphtherin toxin, *Vinca* alkaloids, doxorubicin, methotrexate, *Pseudomonas* endo- and exotoxin or derivatives and pharmaceutically acceptable salts thereof.

20. A pharmaceutical composition comprising an immunoglobulin molecule or monoclonal antibody according to any one of claims 1-19, or a pharmaceutically acceptable derivative thereof and optionally a pharmaceutically acceptable carrier.

21. Use of the composition of claim 20 for the manufacture of a pharmaceutical for the treatment of NCAM-related malignancies and NCAM-related autoimmune disorders in a subject.

22. The use according to claim 21, wherein said subject is a mammal, preferably a human.

23. The use according to any one of claims 21 or 22, wherein the NCAM-related malignancy is selected from the group consisting of neuroblastoma, brain tumor, rhabdomyosarcoma, nephroblastoma (Wilms' tumor), carcinoid, small cell lung cancer, pituitary tumor, acute non-lymphocytic leukemia (ANLL), acute monocytic leukemia (AMoL), acute megakaryocytic leukemia (AMKL), multiple myeloma, myeloid leukemia, insulinoma and large granular lymphocytic leukemia.

24. The use according to any one of claims 21 or 22, wherein the NCAM-related autoimmune disorder is rheumatoid arthritis.

25. The use according to claim 23, further comprising the combined administration with a chemotherapeutic agent.

26. The use according to claim 25, wherein said chemotherapeutic agent is selected from the group of cyclophosphamide, etoposide, vincristine, procarbazine, prednisone, carmustine, doxorubicin, methotrexate, bleomycin, dexamethasone, phenyl butyrate, bryostatin-1 and leucovorin.

27. A hybridoma which secretes anti-NCAM antibody, said hybridoma being identified by deposit number DSM ACC2757.

28. A nucleic acid molecule encoding an immunoglobulin molecule according to any one of claims 1-14.

29. A nucleic acid molecule encoding an immunoglobulin heavy chain or fragment thereof, wherein said immunoglobulin heavy chain or fragment thereof encodes a variable domain comprising the hypervariable regions CDR-H1, CDR-H2, and CDR-H3, wherein said CDR-H1 has the amino acid sequence set forth in SEQ ID NO:12, said CDR-H2 has the amino acid sequence set forth in SEQ ID NO:14, and said CDR-H3 has the amino acid sequence set forth in SEQ ID NO:16.

30. The nucleic acid molecule according to claim 38, wherein said nucleic acid comprises the nucleotide sequences as set forth in SEQ ID NO: 11, SEQ ID NO: 13, and SEQ ID NO: 15.

31. The nucleic acid molecule according to claim 39, wherein said nucleic acid comprises the nucleotide sequence as set forth in SEQ ID NO: 1.

32. A nucleic acid molecule encoding an immunoglobulin light chain or fragment thereof, wherein said immunoglobulin light chain or fragment thereof encodes a variable domain comprising the hypervariable regions CDR-L1, CDR-L2, and CDR-L3, wherein said CDR-L1 has the amino acid sequence set forth in SEQ ID NO: 6, said CDR-L2 has the amino acid sequence set forth in SEQ ID NO: 8, and said CDR-L3 has the amino acid sequence set forth in SEQ ID NO: 10.

33. The nucleic acid molecule according to claim 32, wherein said nucleic acid comprises the nucleotide sequences as set forth in SEQ ID NO: 5, SEQ ID NO: 7, and SEQ ID NO: 9.

34. The nucleic acid molecule according to claim 33, wherein said nucleic acid comprises the nucleotide sequence as set forth in SEQ ID NO: 3.

35. An expression vector comprising any one of the nucleic acid molecules of claims 28-34 and further comprising a promoter region.

36. Use of at least one of the nucleic acid molecules or vectors according to claims 28-35 for producing an immunoglobulin molecule according to any one of claims 1-14, comprising recombinantly expressing the at least one nucleic acid molecule or vector in a cell to obtain said immunoglobulin molecule.

37. The use according to claim 36, wherein said cell is a mammalian cell.

38. Use of a transgenic animal to produce an immunoglobulin molecule according to any one of claims 1-14.

39. Use according to claim 38, wherein said transgenic animal is a mouse, preferably of the F004-Jen strain.

40. A recombinant cell comprising a nucleic acid molecule or vector according to any one of claims 28-34.


SEQUENCE LISTING

<110>  Universitaet Koeln
       Medizinische Hochschule Hannover

<120>  Neural cell adhesion molecule (NCAM) binding molecules

<130>  P201299

<160>  16

<170>  PatentIn version 3.3

<210>  1
<211>  339
<212>  DNA
<213>  Mus musculus

<220>
<221>  CDS
<222>  (1)..(339)

<400>  1
cag gag tct gga cct gag ctg gta aag cct ggg gct tca gtg aag atg      48
Gln Glu Ser Gly Pro Glu Leu Val Lys Pro Gly Ala Ser Val Lys Met
1               5                   10                  15

tcc tgc aag gct tct gga tac aca ttc act agc tat gtt atg gac tgg      96
Ser Cys Lys Ala Ser Gly Tyr Thr Phe Thr Ser Tyr Val Met Asp Trp
            20                  25                  30

gtg aag cag aag cct ggg cag ggc ctt gag tgg att gga tat att aat      144
Val Lys Gln Lys Pro Gly Gln Gly Leu Glu Trp Ile Gly Tyr Ile Asn
        35                  40                  45

cct tac aat gat ggt act aag tac aat gag aag ttc aaa ggc aag gcc      192
Pro Tyr Asn Asp Gly Thr Lys Tyr Asn Glu Lys Phe Lys Gly Lys Ala
    50                  55                  60

aca ctg act tca gac aaa tcc tcc agc aca gcc tac atg gag ctc agc      240
Thr Leu Thr Ser Asp Lys Ser Ser Ser Thr Ala Tyr Met Glu Leu Ser
65                  70                  75                  80

agc ctg acc tct gag gac tct gcg gtc tat tac tgt gca aga tcg ggt      288
Ser Leu Thr Ser Glu Asp Ser Ala Val Tyr Tyr Cys Ala Arg Ser Gly
                85                  90                  95

att acg gac ttt gac tac tgg ggc caa ggc acc act ctc ata gtc tcc      336
Ile Thr Asp Phe Asp Tyr Trp Gly Gln Gly Thr Thr Leu Ile Val Ser
            100                 105                 110

tca                                                                  339
Ser

<210>  2
<211>  113
<212>  PRT

<213>  Mus musculus

<400>  2

Gln Glu Ser Gly Pro Glu Leu Val Lys Pro Gly Ala Ser Val Lys Met
1               5                   10                  15

Ser Cys Lys Ala Ser Gly Tyr Thr Phe Thr Ser Tyr Val Met Asp Trp
            20                  25                  30

Val Lys Gln Lys Pro Gly Gln Gly Leu Glu Trp Ile Gly Tyr Ile Asn
        35                  40                  45

Pro Tyr Asn Asp Gly Thr Lys Tyr Asn Glu Lys Phe Lys Gly Lys Ala
    50                  55                  60

Thr Leu Thr Ser Asp Lys Ser Ser Ser Thr Ala Tyr Met Glu Leu Ser
65                  70                  75                  80

Ser Leu Thr Ser Glu Asp Ser Ala Val Tyr Tyr Cys Ala Arg Ser Gly
                85                  90                  95

Ile Thr Asp Phe Asp Tyr Trp Gly Gln Gly Thr Thr Leu Ile Val Ser
            100                 105                 110

Ser

<210>  3
<211>  316
<212>  DNA
<213>  Mus musculus

<220>
<221>  CDS
<222>  (2)..(316)

<400>  3
c acc cag tct cca gca atc atg tct gca tct cca ggg gag aag gtc tcc      49
  Thr Gln Ser Pro Ala Ile Met Ser Ala Ser Pro Gly Glu Lys Val Ser
  1               5                   10                  15

atg acc tgc agt gcc agc tca agt gta agt tac atg tac tgg ttc caa        97
Met Thr Cys Ser Ala Ser Ser Ser Val Ser Tyr Met Tyr Trp Phe Gln
            20                  25                  30

cag aag cca gga tcc tcc ccc aga ctc ctg att tat gac aca tcc aac       145
Gln Lys Pro Gly Ser Ser Pro Arg Leu Leu Ile Tyr Asp Thr Ser Asn
        35                  40                  45

ctg gct tct gga gtc cct gtt cgc ttc agt ggc agt ggg tct ggg acc       193

Leu Ala Ser Gly Val Pro Val Arg Phe Ser Gly Ser Gly Ser Gly Thr
    50                  55                  60

tct tac tct ctc aca atc agc cga atg gag gct gaa gat gct gcc act        241
Ser Tyr Ser Leu Thr Ile Ser Arg Met Glu Ala Glu Asp Ala Ala Thr
65                  70                  75                  80

tat tac tgc cag cag tgg agt agt tac ccg ctc acg ttc ggt gat ggg        289
Tyr Tyr Cys Gln Gln Trp Ser Ser Tyr Pro Leu Thr Phe Gly Asp Gly
                85                  90                  95

acc aag ctg gag ctg aaa cga act gtg                                    316
Thr Lys Leu Glu Leu Lys Arg Thr Val
            100                 105


<210>  4
<211>  105
<212>  PRT
<213>  Mus musculus

<400>  4

Thr Gln Ser Pro Ala Ile Met Ser Ala Ser Pro Gly Glu Lys Val Ser
1               5                   10                  15


Met Thr Cys Ser Ala Ser Ser Ser Val Ser Tyr Met Tyr Trp Phe Gln
            20                  25                  30


Gln Lys Pro Gly Ser Ser Pro Arg Leu Leu Ile Tyr Asp Thr Ser Asn
        35                  40                  45


Leu Ala Ser Gly Val Pro Val Arg Phe Ser Gly Ser Gly Ser Gly Thr
    50                  55                  60


Ser Tyr Ser Leu Thr Ile Ser Arg Met Glu Ala Glu Asp Ala Ala Thr
65                  70                  75                  80


Tyr Tyr Cys Gln Gln Trp Ser Ser Tyr Pro Leu Thr Phe Gly Asp Gly
                85                  90                  95


Thr Lys Leu Glu Leu Lys Arg Thr Val
            100                 105


<210>  5
<211>  30
<212>  DNA
<213>  Mus musculus


<220>
<221>  CDS
<222>  (1)..(30)

<400>  5
agt gcc agc tca agt gta agt tac atg tac                                30
Ser Ala Ser Ser Ser Val Ser Tyr Met Tyr
1               5                   10


<210>  6
<211>  10
<212>  PRT
<213>  Mus musculus

<400>  6

Ser Ala Ser Ser Ser Val Ser Tyr Met Tyr
1               5                   10


<210>  7
<211>  24
<212>  DNA
<213>  Mus musculus


<220>
<221>  CDS
<222>  (1)..(24)

<400>  7
tat gac aca tcc aac ctg gct tct                                        24
Tyr Asp Thr Ser Asn Leu Ala Ser
1               5


<210>  8
<211>  8
<212>  PRT
<213>  Mus musculus

<400>  8

Tyr Asp Thr Ser Asn Leu Ala Ser
1               5


<210>  9
<211>  27
<212>  DNA
<213>  Mus musculus


<220>
<221>  CDS
<222>  (1)..(27)

<400>  9
cag cag tgg agt agt tac ccg ctc acg                                    27
Gln Gln Trp Ser Ser Tyr Pro Leu Thr
1               5

<210>  10
<211>  9
<212>  PRT
<213>  Mus musculus

<400>  10

Gln Gln Trp Ser Ser Tyr Pro Leu Thr
1               5


<210>  11
<211>  30
<212>  DNA
<213>  Mus musculus


<220>
<221>  CDS
<222>  (1)..(30)

<400>  11
gga tac aca ttc act agc tat gtt atg gac                                30
Gly Tyr Thr Phe Thr Ser Tyr Val Met Asp
1               5                   10


<210>  12
<211>  10
<212>  PRT
<213>  Mus musculus

<400>  12

Gly Tyr Thr Phe Thr Ser Tyr Val Met Asp
1               5                   10


<210>  13
<211>  51
<212>  DNA
<213>  Mus musculus


<220>
<221>  CDS
<222>  (1)..(51)

<400>  13
tat att aat cct tac aat gat ggt act aag tac aat gag aag ttc aaa        48
Tyr Ile Asn Pro Tyr Asn Asp Gly Thr Lys Tyr Asn Glu Lys Phe Lys
1               5                   10                  15

ggc                                                                    51
Gly


<210>  14

<211>  17
<212>  PRT
<213>  Mus musculus

<400>  14

Tyr Ile Asn Pro Tyr Asn Asp Gly Thr Lys Tyr Asn Glu Lys Phe Lys
1               5                   10                  15

Gly

<210>  15
<211>  24
<212>  DNA
<213>  Mus musculus

<220>
<221>  CDS
<222>  (1)..(24)

<400>  15
tcg ggt att acg gac ttt gac tac                                          24
Ser Gly Ile Thr Asp Phe Asp Tyr
1               5

<210>  16
<211>  8
<212>  PRT
<213>  Mus musculus

<400>  16

Ser Gly Ile Thr Asp Phe Asp Tyr
1               5